# EUROPÄISCHE PATENTSCHRIFT

(11) **EP 1 472 201 B1**
(45) Veröffentlichungstag und Bekanntmachung des Hinweises auf die Patenterteilung: **13.08.2014**
(21) Anmeldenummer: 03702479.1
(22) Anmeldetag: 21.01.2003
(51) Int. Cl.: C07F 9/46, C07F 9/48, C07F 9/52, C07F 9/572, C07F 9/6503, C07F 9/6574, C07F 9/6584, C07C 45/50, C07C 403/12, C07F 9/142, C07F 9/145

(54) **VERFAHREN ZUR ABTRENNUNG VON SÄUREN AUS CHEMISCHEN REAKTIONS GEMISCHEN MIT HILFE VON IONISCHEN FLÜSSIGKEITEN**
METHOD FOR THE SEPARATION OF ACIDS FROM CHEMICAL REACTION MIXTURES BY MEANS OF IONIC FLUIDS
PROCÉDÉ DE SÉPARATION D'ACIDES À PARTIR DE MÉLANGES RÉACTIONNELS AU MOYEN DE LIQUIDES IONIQUES

(30) Priorität: 24.01.2002 DE 10202838; 04.07.2002 DE 10230222; 18.10.2002 DE 10248902; 31.10.2002 DE 10251140
(43) Veröffentlichungstag der Anmeldung: 03.11.2004
(73) Patentinhaber: BASF SE, 67056 Ludwigshafen (DE)
(72) Erfinder: MAASE, Matthias, 67346 Speyer (DE); MASSONNE, Klemens, 67098 Bad Dürkheim (DE); HALBRITTER, Klaus, 69124 Heidelberg (DE); NOE, Ralf, 68163 Mannheim (DE); BARTSCH, Michael, 67433 Neustadt (DE); SIEGEL, Wolfgang, 67117 Limburgerhof (DE); STEGMANN, Veit, 68165 Mannheim (DE); FLORES, Miguel, 28300 Aranjuez (Madrid) (DE); HUTTENLOCH, Oliver, 67063 Ludwigshafen (DE); BECKER, Michael, 67063 Ludwigshafen (DE)
(86) Internationale Anmeldenummer: PCT/EP2003/000545
(87) Internationale Veröffentlichungsnummer: WO 2003/062171

(56) Entgegenhaltungen:
- EP-A- 1 142 898
- EP-A- 1 142 898
- WO-A-00/56451
- WO-A-01/58589
- WO-A-02/00669
- WO-A-02/05955
- WO-A-02/22261
- WO-A-99/65606
- WO-A-02/083695
- DE-A- 19 724 884
- DE-A- 19 724 884
- DE-A- 19 826 936
- DE-A- 19 826 936
- DE-A1- 10 005 794
- DE-A1- 10 052 462
- DE-A1- 19 725 796
- DE-A1- 19 824 121
- FR-A- 2 810 666
- FR-A- 2 810 666
- US-A- 6 048 997

## Beschreibung

Die vorliegende Erfindung beschreibt ein Verfahren zur vereinfachten Abtrennung von Säuren aus Reaktionsgemischen mittels einer ionischen Flüssigkeit.

Der chemische Fachmann hat oft das Problem, während einer chemischen Reaktion freigesetzte Säuren abzufangen oder Säuren aus Reaktionsgemischen abzutrennen. Beispiele für Reaktionen, in denen Säuren im Reaktionsverlauf freigesetzt werden sind die Silylierung von Alkoholen oder Aminen mit Halogensilanen, die Phosphorylierung von Aminen oder Alkoholen mit Phosphorhalogeniden, die Bildung von Sulfonsäureestern oder-amiden aus Alkoholen oder Aminen und Sulfonsäurechloriden oder-anhydriden, Eliminierungen oder Substitutionen.

Bei diesen Reaktionen werden Säuren freigesetzt, weshalb zusätzlich eine Hilfsbase zugesetzt wird, die in der Regel nicht als Reaktant an der eigentlichen Reaktion teilnimmt. In der Regel ist es notwendig, die freigesetzten Säuren mit dieser Base unter Salzbildung zu binden, um Neben- und Folgereaktionen zu unterbinden oder aber einfach um die Säure aus dem gewünschten Reaktionsprodukt zu entfernen und ggf. in den Prozeß zurückzuführen. Werden die Salze der verwendeten Basen zunächst nicht abgetrennt, so können sie auch in Gegenwart des Wertproduktes, z.B. durch Zugabe einer weiteren, stärkeren Base, wie wäßrigen Laugen, z.B. Natronlauge oder Kalilauge, aufgearbeitet werden. Dabei entsteht das Salz der in diesem Schritt hinzugefügten stärkeren Base. Außerdem wird die ursprünglich verwendete Base in Freiheit gesetzt. Diese beiden Komponenten, d.h. das Salz der stärkeren Base und die in Freiheit gesetzte zuerst verwendete Base (Hilfsbase) müssen in aller Regel ebenfalls vom Wertprodukt abgetrennt
werden. Bei dieser Vorgehensweise ist es oft von Nachteil, daß das Wertprodukt, das bei der Aufarbeitung zugegen ist, durch die hinzugefügte stärkere Base selbst oder weitere Stoffe in dieser Base, z.B. dem Wasser in einer wässrigen Lauge, zersetzt werden kann.

Die Salze der Hilfsbase mit der Säure sind in der Regel in organischen Lösemitteln nicht löslich und weisen hohe Schmelzpunkte auf, so daß sie in organischen Medien Suspensionen bilden, die schwieriger zu handhaben sind als beispielsweise Flüssigkeiten. Es wäre also wünschenswert, die Salze der Hilfsbasen in flüssiger Form abtrennen zu können. Zudem würden die bekannten verfahrenstechnischen Nachteile von Suspensionen eliminiert. Diese sind z.B. die Bildung von Verkrustungen, Verringerung des Wärmeüberganges, schlechte Durchmischung und Rührbarkeit sowie die Bildung von lokalen Über- oder Unterkonzentrationen und sogenannten hot spots.

Der Stand der Technik beeinhaltet für industriell durchgeführte Verfahren demnach folgende Nachteile:
1) Zugabe von zwei Hilfsstoffen, der Hilfsbase sowie einer weiteren starken Base und der daraus erwachsenden Aufgabe, zwei Hilfsstoffe vom Wertprodukt und voneinander abzutrennen,
2) Handhabung von Suspensionen
3) Abtrennung des Salzes der starken Base als Feststoff.

Anzustreben ist jedoch eine verfahrenstechnisch einfache Phasentrennung mittels einer flüssig-flüssig-Phasentrennung.

Aus DE-A 197 24 884 und DE-A 198 26 936 sind Verfahren zur Herstellung von Carbonyldiimidazolen durch Phosgenierung von Imidazolen bekannt, bei denen das gebildete Hydrochlorid des als Edukt eingesetzten Imidazols als Schmelze aus dem Reaktionsgemisch abgetrennt wird. In der DE-A 198 26 936 wird auf S. 3, Zeile 5 darauf hingewiesen, daß das Hydrochlorid des Imidazols überraschenderweise bei Temperaturen von 110-130°C flüssig ist und bereits deutlich unterhalb des in der Literatur beschriebenen Schmelzpunktes von 158-161 °C schmilzt. Als Grund dafür geben die Erfinder entweder die Bildung eines eutektischen Gemisches aus dem Imidazol Hydrochlorid mit dem Wertprodukt Carbonyldiimidazol oder die Bildung eines ternären Gemisches aus dem Imidazol Hydrochlorid, dem Wertprodukt Carbonyldiimidazol und Lösemittel Chlorbenzol an. Obwohl das Imidazol Hydrochlorid nicht hätte flüssig vorliegen dürfen, war das in diesem speziellen Fall doch überraschenderweise der Fall. Die Anwendbarkeit dieses Konzepts für andere Reaktionen als die Phosgenierung von Imidazolen wird nicht beschrieben.

Es bestand also die Aufgabe, auch für andere chemische Reaktionen oder für die Abtrennung von Säuren, die in Gemischen enthalten sind, aber nicht während einer chemischen Reaktion abgespalten werden, ein vereinfachtes Verfahren zur Abtrennung von Säuren zu finden, bei dem ein aus einer eingesetzten Hilfsbase und einer Säure gebildetes Salz über eine verfahrenstechnisch einfache flüssig-flüssig-Phasentrennung abgetrennt werden kann.

Die Aufgabe wurde gelöst durch ein Verfahren zur Abtrennung von Säuren aus Reaktionsgemischen mittels einer Hilfsbase, welches dadurch gekennzeichnet ist, daß die Hilfsbase
b) ein Salz mit der Säure bildet, das bei Temperaturen flüssig ist, bei denen das Wert-produkt während der Abtrennung des Flüssigsalzes nicht zersetzt wird und
c) das Salz der Hilfsbase mit dem Wertprodukt oder der Lösung des Wertproduktes in einem geeigneten Lösungsmittel zwei nicht mischbare flüssige Phasen ausbildet, wobei die Säure im Reaktionsverlauf einer Phosphorylierung zur Herstellung von Phosphinen, Phosphinsäureestern, Phosphinigsäureestern (Phosphiniten), Phosphonsäureestern, Phosphonsäurehalogeniden, Phosphonsäureamiden, Phosphonigsäureestern (Phosphoniten), Phosphonigsäureamiden. Phosphonigsäurehalogeniden, Phosphorsäureestern, Phosphorsäurediesterhalogeniden, Phosphorsäurediesteramiden, Phosphorsäureesterdihalogeniden, Phosphorsäureesterdiamiden, Phosphorigsäureesten

Dem Fachmann ist bekannt, daß die Abtrennung einer flüssigen Phase von einer zweiten flüssigen Phase verfahrenstechnisch erheblich einfacher zu gestalten ist als eine Feststoffabtrennung.

Der technische Nutzen des erfindungsgemäßen Verfahrens besteht darin, daß die Abtrennung des Hilfsstoffes durch eine einfache Flüssig-Flüssig-Phasentrennung erfolgen kann, so daß der verfahrenstechnisch aufwendige Umgang mit Feststoffen wegfällt.

Die Aufarbeitung der Hilfsstoffe kann in Abwesenheit des Wertproduktes erfolgen, so daß letzteres weniger belastet wird.

Nicht mischbar bedeutet, daß sich mindestens zwei, durch eine Phasengrenzfläche getrennte flüssige Phasen ausbilden.

Wenn das reine Wertprodukt mit dem Salz aus der Hilfsbase und der Säure gänzlich oder zu einem größeren Teil mischbar ist, kann dem Wertprodukt auch ein Hilfsstoff, z.B. ein Lösemittel zugesetzt werden, um eine Entmischung oder Löslichkeitsverringerung zu erreichen. Dies ist beispielsweise dann sinnvoll, wenn die Löslichkeit des Salzes im Wertprodukt oder umgekehrt 20 Gew.-% oder mehr beträgt, bevorzugt 15 Gew.-% oder mehr, besonders bevorzugt 10 Gew-% oder mehr und ganz besonders bevorzugt 5 Gew.-% oder mehr beträgt. Die Löslichkeit wird bestimmt unter den Bedingungen der jeweiligen Trennung. Bevorzugt wird die Löslichkeit bestimmt bei einer Temperatur, die oberhalb des Schmelzpunktes des Salzes liegt und unterhalb der niedrigsten der folgenden Temperaturen, besonders bevorzugt 10 °C unterhalb der niedrigsten und ganz besonders bevorzugt 20 °C unterhalb der niedrigsten:
- Siedepunkt des Wertproduktes
- Siedepunkt des Lösemittels
- Temperatur der signifikanten Zersetzung des Wertproduktes

Das Lösungsmittel ist dann als geeignet anzusehen, wenn das Gemisch aus Wertprodukt und Lösungsmittel das Salz bzw. das Salz das Wertprodukt oder eine Mischung aus Wertprodukt und Lösemittel weniger als die oben angegebenen Mengen zu lösen vermag. Als Lösungsmittel verwendbar sind beispielsweise Benzol, Toluol, o-, m- oder p-Xylol, Cyclohexan, Cyclopentan, Pentan, Hexan, Heptan, Oktan, Petrolether, Aceton, Isobutylmethylketon, Diethylketon, Diethylether, *tert*.-Butylmethylether, *tert*.-Butylethylether, Tetrahydrofuran, Dioxan, Essigester, Methylacetat, Dimethylformamid, Dimethylsulfoxid, Acetonitril, Chloroform, Dichlormethan, Methylchloroform oder Gemische davon.

Bei dem Wertprodukt handelt es sich in der Regel um unpolare organische oder anorganische Verbindungen.

Reaktionen, bei denen das Verfahren angewendet werden kann sind
- Phosphorylierungen, also Umsetzungen mit Verbindungen, die mindestens eine P-Hal-Bindung enthalten, wie z.B. PCl₃, PCl₅, POCl₃, POBr₃, Dichlorphenylphosphin oder Diphenylchlorphosphin, wie sie beispielsweise ebenfalls von Chojnowski et al., a.a.O. beschrieben sind,

Die abzutrennenden Säuren können Brönstedsäuren und Lewis-Säuren sein. Welche Säuren als Brönsted und Lewissäuren bezeichnet werden, wird in Hollemann-Wiberg, Lehrbuch der Anorganischen Chemie, 91.-100. Auflage, Walter de Gruyter, Berlin New York 1985, S. 235 bzw. S. 239 beschrieben. Zu den Lewissäuren im Sinne dieser Erfindung zählen auch die als Friedel-Crafts-Katalysatoren verwendeten Lewissäuren, die in George A. Olah, Friedel-Crafts an Related Reactions, Vol. I, 191 bis 197, 201 und 284-90 (1963) beschrieben sind. Als Beispiele genannt seien Aluminiumtrichlorid (AlCl₃), Eisen(III)chlorid (FeCl₃), Aluminiumtribromid (AlBr₃) und Zinkchlorid (ZnCl₂).

Allgemein enthalten die abtrennbaren Lewis-Säuren kationische Formen der Metalle der Gruppen Ib, IIb, IIIa, IIIb, IVa, IVb, Va, Vb, Vlb, VIIb und VIII des Periodensystems der Elemente sowie der seltenen Erden, wie beispielsweise Lanthan, Cer, Praseodym, Neodym, Samarium, Europium, Gadolinium, Terbium, Dysprosium, Holmium, Erbium, Thulium, Ytterbium oder Lutetium.

Genannt seien besonders Zink, Cadmium, Beryllium, Bor, Aluminium, Gallium, Indium, Thallium, Titan, Zirkon, Hafnium, Erbium, Germanium, Zinn, Vanadium, Niob, Skandium, Yttrium, Chrom, Molybden, Wolfram, Mangan, Rhenium, Palladium, Thorium, Eisen, Kupfer und Kobalt. Bevorzugt sind Bor, Zink, Cadmium, Titan, Zinn, Eisen, Kobalt.

Als Gegenionen der Lewis-Säure kommen in Frage F⁻, Cl⁻, ClO⁻, ClO₃⁻, ClO₄⁻, Br⁻, J⁻, JO₃⁻, CN⁻, OCN⁻, SCN⁻, NO₂⁻, NO₃⁻, HCO₃⁻, CO₃²⁻, S²-, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, Dithiocarbamat, Salicylat, (OCₙH₂ₙ₊₁)~, (CₙH₂ₙ₋₁O₂)⁻, (CₙH₂ₙ₋₃O₂)⁻ sowie (Cₙ₊₁H₂ₙ₋₂O₄)²⁻, wobei n für die Zahlen 1 bis 20 steht, Methansulfonat (CH₃SO₃⁻), Trifluormethansulfonat (CF₃SO₃⁻), Toluolsulfonat (CH₃C₆H₄SO₃⁻), Benzolsulfonat (C₆H₅SO₃⁻), Hydroxid (OH⁻), Anionen aromatischer Säuren wie Benzoesäure, Phtalsäure, und dergleichen und 1,3-Dicarbonylverbindungen.

Weiterhin genannt seien Carboxylate, insbesondere sind zu erwähnen Formiat, Acetat, Trifluoracetat, Propionat, Hexanoat und 2-Ethylhexanoat, Stearat sowie Oxalat, Acetylacetonat, Tartrat, Acrylat und Methacrylat, bevorzugt Formiat, Acetat, Propionat, Oxalat, Acetylacetonat, Acrylat und Methacrylat.

Weiterhin kommen Borhydride und Organoborverbindungen der allgemeinen Formel BR""₃ und B(OR"")₃ in Betracht, worin R"" jeweils unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein kann. Die Reste R"" können auch miteinander verbunden sein.

Als bevorzugte Beispiele für Lewis-Säuren seien neben den oben angeführten AlCl₃, FeCl₃, AlBr₃ und ZnCl₂ genannt BeCl₂, ZnBr₂, ZnI₂, ZnSO₄, CuCl₂, CuCl, Cu(O₃SCF₃)₂, CoCl₂, CoI₂, FeI₂, FeCl₂, FeCl₂(THF)₂, TiCl₄(THF)₂, TiCl₄, TiCl₃, ClTi(OiPr)₃, SnCl₂, SnCl₄, Sn(SO₄), Sn(SO₄)₂, MnCl₂, MnBr₂, ScCl₃, BPh₃, BCl₃, BBr₃, BF₃•OEt₂, BF₃•OMe₂, BF₃•MeOH, BF₃•CH₃COOH, BF₃•CH₃CN, B(CF₃COO)₃, B(OEt)₃, B(OMe)₃, B(OiPr)₃, PhB(OH)₂, 3-MeO-PhB(OH)₂, 4-MeO-PhB(OH)₂, 3-F-PhB(OH)₂, 4-F-PhB(OH)₂, (C₂H₅)Al, (C₂H₅)₂AlCl, (C₂H₅)AlCl₂, (C₈H₁₈)AlCl₂, (C₈H₁₇)₂AlCl, (iso-C₄H₉)₂AlCl, Ph₂AlCl, PhAlCl₂, Al(acac)₃, Al(OiPr)₃, Al(OnBu)₃, Al(OsekBu)₃, Al(OEt)₃, GaCl₃, ReCl₅, ZrCl₄, NbCl₅, VCl₃, CrCl₂, MoCl₅, YCl₃, CdCl₂, CdBr₂, SbCl₃, SbCl₅, BiCl₃, ZrCl₄, UCl₄, LaCl₃, CeCl₃, Er(O₃SCF₃), Yb(O₂CCF₃)₃, SmCl₃, SmI₂, B(C₆H₅)₃, TaCl₅. Die Lewis-Säuren können stabilisiert sein durch Alkali- oder Erdalkalimetallhalogenide, beispielsweise LiCl oder NaCl. Dazu werden die (Erd)Alkalimetallhalogenide zur Lewis-Säure im molaren Verhältnis 0 - 100 : 1 gemischt.

Mit Halogen oder Hal ist im Rahmen dieser Schrift Fluor (F), Chlor (Cl), Brom (Br) oder Iod (I), bevorzugt Chlor gemeint.

Umgesetzt im Sinne einer Phosphorylierung werden in der Regel Verbindungen, die mindestens eine freie O-H-, S-H- oder N-H-Bindung aufweisen, gegebenenfalls nach Deprotonierung durch die Hilfsbase.

Bevorzugt sind solche Hilfsbasen, die nicht als Reaktant an der Reaktion teilnehmen.

Weiterhin bevorzugt kann diese Hilfsbase zusätzlich gleichzeitig als nucleophiler Katalysator in der Reaktion fungieren, d.h. sie erhöht die Reaktionsgeschwindigkeit der Reaktion gegenüber der Durchführung ohne Anwesenheit einer Hilfsbase um das mindestens 1,5-fache, bevorzugt um das mindestens zweifache, besonders bevorzugt um das fünf-fache, ganz besonders bevorzugt um das mindestens zehnfache und insbesondere um das mindestens zwanzigfache.

Erfindungsgemäß wird eine Hilfsbase der Formel (Ie) eingesetzt, 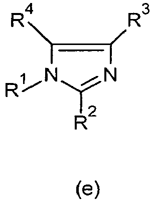 worin
R¹ ausgewählt ist unter Methyl, Ethyl, n-Propyl, n-Butyl, n-Pentyl, n-Octyl, 2-Hydroxyethyl oder 2-Cyanoethyl und
R² bis R⁴ unabhängig voneinander Wasserstoff, Methyl oder Ethyl bedeuten.

Insbesondere bevorzugt sind 1-n-Butylimidazol, 1-Methylimidazol.

Säuren, mit denen die Basen Salze bilden können sind beispielsweise lodwasserstoffsäure (HI), Fluorwasserstoff (HF), Chlorwasserstoff (HCl), Salpetersäure (HNO₃), salpetrige Säure (HNO₂), Bromwasserstoffsäure (HBr), Kohlensäure (H₂CO₃), Hydrogencarbonat (HCO₃⁻), Methylkohlensäure (HO(CO)OCH₃), Ethylkohlensäure (HO(CO)OC₂H₅), n-Butylkohlensäure, Schwefelsäure (H₂SO₄), Hydrogensulfat (HSO₄⁻), Methylschwefelsäure (HO(SO₂)OCH₃), Ethylschwefelsäure (HO(SO₂)OC₂H₅), Phosphorsäure (H₃PO₄), Dihydrogenphosphat (H₂PO₄⁻), Ameisensäure (HCOOH), Essigsäure (CH₃COOH), Propionsäure, n- und iso-Buttersäure, Pivalinsäure, para-Toluolsulfonsäure, Benzolsulfonsäure, Benzoesäure, 2,4,6-Trimethylbenzoesäure, Mandelsäure, Methansulfonsäure, Ethansulfonsäure oder Trifluormethansulfonsäure, bevorzugt sind Chlorwasserstoff, Essigsäure, p-Toluolsulfonsäure, Methansulfonsäure, 2,4,6-Trimethylbenzoesäure und Trifluormethansulfonsäure und besonders bevorzugt ist Chlorwasserstoff.

In einer bevorzugten Ausführungsform zur Abtrennung von Brönsted-Säuren (Protonensäuren) werden diese ohne große Anteile von Lewis-Säuren abgetrennt, d.h. im abgetrennten Salz der Säure mit der Hilfsbase ist das molare Verhältnis von Brönsted-Säuren zu Lewis-Säuren größer als 4:1, bevorzugt größer als 5:1, besonders bevorzugt größer als 7:1, ganz besonders bevorzugt größer als 9:1 und insbesondere größer als 20:1.

Bevorzugt sind solche Hilfsbasen, deren Salze aus Hilfsbasen und Säuren, eine Schmelztemperatur aufweisen, bei der im Zuge der Abtrennung des Salzes als flüssige Phase keine signifikante Zersetzung des Wertproduktes auftritt, d.h. weniger als 10 Mol% pro Stunde, bevorzugt weniger als 5 Mol %/h, besonders bevorzugt weniger als 2 Mol %/h und ganz besonders bevorzugt weniger als 1 Mol %/h.

Die Schmelzpunkte der Salze der besonders bevorzugten Hilfsbasen liegen in der Regel unterhalb von 160°-C, besonders bevorzugt unterhalb von 100°-C und ganz besonders bevorzugt unterhalb von 80°C.

Unter den Hilfsbasen sind solche ganz besonders bevorzugt, deren Salze einen E_{T}(30)-Wert von > 35, bevorzugt von >40, besonders bevorzugt von > 42 aufweisen. Der E_{T}(30)-Wert ist ein Maß für die Polarität und wird von C. Reichardt in Reichardt, Christian Solvent Effects in Organic Chemistry Weinheim : VCH, 1979. - XI, (Monographs in Modern Chemistry ; 3), ISBN 3-527-25793-4 Seite 241 beschrieben.

Eine außergewöhnlich bevorzugte Base, welche die Aufgabenstellung z.B. erfüllt, ist 1-Methylimidazol. Die Verwendung von 1-Methylimidazol als Base wird z.B. in DE-A 35 02 106 erwähnt, jedoch wird dort nicht deren Verwendbarkeit als ionische Flüssigkeit erkannt.

1-Methylimidazol ist zudem noch als nucleophiler Katalysator wirksam [Julian Chojnowski, Marek Cypryk, Witold Fortuniak, Heteroatom. Chemistry, 1991, 2, 63-70]. Chojnowski et al. haben gefunden, daß 1-Methylimidazol im Vergleich zu Triethylamin die Phosphorylierung von t-Butanol um den Faktor 33 und die Silylierung von Pentamethyldisiloxanol um den Faktor 930 beschleunigt.

Es wurde weiterhin gefunden, daß das Hydrochlorid von 1-Methylimidazol einen Schmelzpunkt von etwa 75°C aufweist und mit unpolaren organischen Wertprodukten, wie z.B. Diethoxyphenylphosphin, Triethylphosphit, Ethoxydiphenylphosphin, Alkylketendimer, Alkoxysilane oder Ester, oder Lösemitteln im wesentlichen nicht mischbar ist. So bildet 1-Methylimidazol•HCl im Gegensatz zu dem polaren Lösemittel Wasser sogar mit Aceton zwei nicht mischbare Phasen aus. 1-Methylimidazol kann zugleich als Hilfsbase und nucleophiler Katalysator dienen und als flüssiges Hydrochlorid über eine verfahrenstechnisch einfache Flüssig-Flüssig-Phasentrennung von organischen Medien abgetrennt werden.

Statt 1-Methylimidazol kann auch 1-Butylimidazol verwendet werden. Das Hydrochlorid des 1-Butylimidazols ist bereits bei Raumtemperatur flüssig, so daß 1-Butylimidazol als Hilfsbase und Katalysator für Reaktionen verwendet werden kann, bei denen Stoffe gehandhabt werden, die bei Temperaturen oberhalb der Raumtemperatur bereits zersetzlich sind. Ebenfalls bei Raumtemperatur flüssig ist das Acetat und Formiat von 1-Methylimidazol.

Ebenso können alle Derivate des Imidazols verwendet werden, deren Salze einen E_{T}(30)-Wert von > 35, bevorzugt von >40, besonders bevorzugt von > 42 aufweisen und eine Schmelztemperatur haben, bei der im Zuge der Abtrennung des Salzes als flüssige Phase keine signifikante Zersetzung des Wertproduktes auftritt. Die polaren Salze dieser Imidazole bilden wie oben angeführt mit weniger polaren organischen Medien zwei nicht mischbare Phasen aus.

Die Durchführung der Reaktion ist nicht beschränkt und kann erfindungsgemäß unter Abfangen der freigesetzten oder zugesetzten Säuren, gegebenenfalls unter nucleophiler Katalyse, diskontinuierlich oder kontinuierlich und an Luft oder unter einer Schutzgasatmosphäre durchgeführt werden.

Bei temperaturempfindlichen Wertprodukten kann es ausreichend sein, das Salz aus Hilfsbase und Säure als festes Salz während der Reaktion ausfallen zu lassen und erst zur Aufarbeitung oder nach Abtrennung der Hauptmenge des Wertproduktes in einer fest-flüssig-Trennung aufzuschmelzen. Das Produkt wird dadurch thermisch weniger belastet.

Ein weiterer Gegenstand der Erfindung ist ein Verfahren zur Abtrennung der oben angeführten Hilfsbasen oder Hilfsbasen, die als nucleophile Katalysatoren verwendet werden, aus einem Reaktionsgemisch, indem man das Reaktionsgemisch pro mol Hilfsbase mit mindestens einem mol Säure versetzt. Dadurch wird die Abtrennung solcher Hilfsbasen als ionische Flüssigkeiten mit Hilfe einer flüssig-flüssig-Trennung möglich.

Aus dem vom Wertprodukt abgetrennten Salz der Hilfsbase kann nach dem Fachmann bekannter Art und Weise die freie Base wiedergewonnen und in den Prozeß zurückgeführt werden.

Dies kann beispielsweise erfolgen, indem man das Salz der Hilfsbase mit einer starken Base, z.B. NaOH, KOH, Ca(OH)₂, Kalkmilch, Na₂CO₃, NaHCO₃, K₂CO₃, oder KHCO₃, gegebenenfalls in einem Lösungsmittel, wie z.B. Wasser, Methanol, Ethanol, n- oder iso-Propanol, n-Butanol, n-Pentanol oder Butanol- oder Pentanol-Isomerengemische oder Aceton, freisetzt. Die so freigesetzte Hilfsbase kann , wenn sie eine eigene Phase ausbildet abgetrennt oder falls sie mit dem Salz der stärkeren Base bzw. der Lösung des Salzes der stärkeren Base mischbar ist, durch Destillation aus der Mischung abgetrennt werden. Falls erforderlich kann man die freigesetzte Hilfsbase auch vom Salz der stärkeren Base bzw. der Lösung des Salzes der stärkeren Base durch Extraktion mit einem Extraktionsmittel abtrennen. Extraktionsmittel sind z.B. Lösemittel, Alkohole oder Amine.

Falls erforderlich kann die Hilfsbase mit Wasser oder wäßriger NaCl oder Na₂SO₄-Lösung gewaschen und anschließend getrocknet werden, z.B. durch Abtrennung von gegebenenfalls enthaltenem Wasser mit Hilfe einer Azeotropdestillation mit Benzol, Toluol, Xylol Butanol oder Cyclohexan.

Falls erforderlich, kann die Base vor erneuter Verwendung destilliert werden.

Eine weitere Möglichkeit der Rückführung ist, das Salz der Hilfsbase zu destillieren, wobei das Salz thermisch in seine Ausgangsstoffe, d.h. die freie Base und die abgefangene Säure gespalten wird. Der leichter siedende Anteil des Salzes wird abdestilliert, während der höhersiedende im Sumpf verbleibt. Die freie Hilfsbase ist dabei entweder der Leicht- oder Hochsieder. Auf diese Weise kann z.B. 1-Butylimidazolformiat destillativ in Ameisensäure (Kopfprodukt) und 1-Butylimidazol (Sumpfprodukt) getrennt werden, wie in der EP-A 181 078 beschrieben.

Mit dem Verfahren durchführbare Phosphorylierungen sind solche Reaktionen, bei denen Phosphorverbindungen, beispielsweise Phosphine, Phosphinsäureester, Phosphinigsäureester (Phosphinite), Phosphonsäureester, Phosphonsäurehalogenide, Phosphonsäureamide, Phosphonigsäureester (Phosphonite), Phosphonigsäureamide, Phosphonigsäurehalogenide, Phosphorsäureester, Phosphorsäurediesterhalogenide, Phosphorsäurediesteramide, Phosphorsäureesterdihalogenide, Phosphorsäureesterdiamide, Phosphorigsäureester (Phosphite), Phosphorigsäurediesterhalogenide, Phosphorigsäurediesteramide, Phosphorigsäureesterdihalogenide gebildet werden und im Reaktionsverlauf eine Säure abgespalten wird, die mit der Hilfsbase ein Salz bildet wie oben beschrieben.

| | |
|---|---|
| 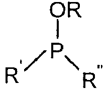 | 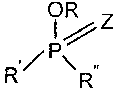 |
| Phosphinigsäureester = Phosphinite | Phosphinsäureester |
| 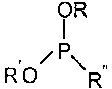 | 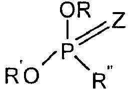 |
| Phosphonigsäureester = Phosphonite | Phosphonsäureester |
| 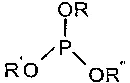 | 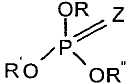 |
| Phosphorigsäureester = Phosphite | Phosphorsäureester |
| 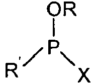 | 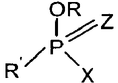 |
| Phosphonigsäureesterhalogenide -bzw. amide | Phosphonsäureesterhalogenid bzw. -amid |
| 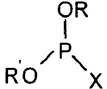 | 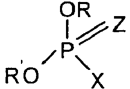 |
| Phosphorigsäurediesterhalogenid bzw. -amid | Phosphorsäurediesterhalogenid bzw. -amid |
| 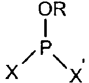 | 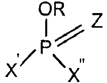 |
| Phosphorigsäureesterdihalogenid | Phosphorsäureesterdihologenid |

Darin stehen R, R' und R" für beliebige Reste, X und X' für Halogen oder Pseudohalogen, wie beispielsweise F, Cl, Br, I, CN, OCN oder SCN oder un-, mono- oder disubstituierte Aminogruppen und Z für Sauerstoff, Schwefel oder ein un- oder monosubstituiertes Stickstoffatom.

Dabei kann es sich um Phosphorverbindungen handeln, die ein oder mehrere, beispielsweise zwei, drei oder vier, bevorzugt zwei oder drei, besonders bevorzugt zwei Phosphoratome aufweisen. In solchen Verbindungen sind die Phosphoratome typischerweise durch ein Brücke verbunden.

Beispielsweise können solche verbrückten Verbindungen mit zwei Phosphoratomen sein:
Diphosphite

   (RO)(R'O)P-O-Z-O-P(OR")(OR"') (Formel II),
Diphosphonite

   (RO)R'P-O-Z-O-PR"(OR"') (Formel III),
Diphosphinite

   (R)(R')P-O-Z-O-P(R")(R"') (Formel IV),
Phosphit-Phosphonite

   (RO)(R'O)P-O-Z-O-P(OR")(R"') (Formel V),
Phosphit-Phosphinite

   (RO)(R'O)P-O-Z-O-P(R")(R"') (Formel VI),
Phosphonit-Phosphinite

   (R)(R'O)P-O-Z-O-P(R")(R"') (Formel VII),

Darin können R, R', R" und R'" beliebige organische Reste und Z eine beliebige bivalente Brücke sein.

Dabei kann es sich jeweils und unabhängig voneinander beispielsweise um ein bis 20 Kohlenstoffatome aufweisende, lineare oder verzweigte, substituierte oder unsubstituierte, aromatische oder aliphatische Reste, wie C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, handeln.

Die genannten Verbindungen können jeweils symmetrisch oder unsymmetrisch substituiert sein.

Phosphorverbindungen mit einem Phosphoratom sind beispielsweise solche der Formel (VIII)

P (X'R⁷) (X²R⁸) (X³R⁹) (VIII)

mit
X¹, X², X³ unabhängig voneinander Sauerstoff, Schwefel, NR¹⁰ oder Einzelbindung
R⁷, R⁸, R⁹, R¹⁰ unabhängig voneinander gleiche oder unterschiedliche organische Reste.

Phosphorverbindungen mit zwei Phosphoratomen sind beispielsweise solche der Formel (IX) 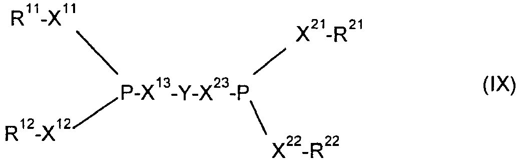 mit
X¹¹, X¹², X¹³, X²¹, X²², X²³ unabhängig voneinander Sauerstoff, Schwefel, NR¹⁰ oder Einzelbindung
R¹¹, R¹² unabhängig voneinander gleiche oder unterschiedliche, einzelne oder verbrückte organische Reste
R²¹, R²² unabhängig voneinander gleiche oder unterschiedliche, einzelne oder verbrückte organische Reste,
Y Brückengruppe.

Die beschriebenen Phosphorverbindungen sind beispielsweise als Liganden für Katalysatoren für die Hydrocyanierung von Butadien zu einem Gemisch isomerer Pentennitrile geeignet. Neben der Hydrocyanierung von 1,3-Butadien-haltigen Kohlenwasserstoffgemischen eignen sich die Katalysatoren im Allgemeinen für alle gängigen Hydrocyanierungsverfahren. Dabei sei insbesondere die Hydrocyanierung von nichtaktivierten Olefinen, z. B. von Styrol und 3-Pentennitril, genannt. Weiterhin ist der Einsatz zur Hydrierung, Hydroformylierung, Hydrocarboxylierung, Hydroamidierung, Hydroveresterung und Aldolkondensation denkbar.

Derartige Katalysatoren können einen oder mehrere der Phosphorverbindungen als Liganden aufweisen. Zusätzlich zu den Phosphorverbindungen als Liganden können sie noch wenigstens einen weiteren Liganden, der ausgewählt ist unter Cyanid, Halogeniden, Aminen, Carboxylaten, Acetylaceton, Aryl- oder Alkylsulfonaten, Hydrid, CO, Olefinen, Dienen, Cycloolefinen, Nitrilen, Nhaltigen Heterocyclen, Aromaten und Heteroaromaten, Ethern, PF₃ sowie ein, zwei- und mehrzähnigen Phosphin-, Phosphinit-, Phosphonit- und Phosphitliganden aufweisen. Diese weiteren Liganden können ebenfalls ein, zwei- oder mehrzähnig sein und an das Metall koordinieren. Geeignete weitere phosphorhaltige Liganden sind z. B. die zuvor als Stand der Technik beschriebenen Phosphin-, Phosphinit- und Phosphitliganden.

Bevorzugt handelt es sich bei dem Metall um eines der VIII. Nebengruppe, besonders bevorzugt um Cobalt-, Rhodium-, Ruthenium-, Palladium- oder Nickelatome in beliebigen Oxidationsstufen. Werden die erfindungsgemäßen Katalysatoren zur Hydrocyanierung eingesetzt, so handelt es sich bei dem Metall der VIII. Nebengruppe insbesondere um Nickel.

Setzt man Nickel ein, so kann dieses in verschiedenen Wertigkeiten, wie 0, +1, +2, +3, vorliegen. Bevorzugt ist hierbei Nickel(0) und Nickel(+2), insbesondere Nickel(0).

Bei Katalysatoren für Hydroformylierungen werden im Allgemeinen unter Hydroformylierungsbedingungen aus den jeweils eingesetzten Katalysatoren oder Katalysatorvorstufen katalytisch aktive Spezies gebildet.

Hierfür wird als Metall vorzugsweise Cobalt, Ruthenium, Rhodium, Palladium, Platin, Osmium oder Iridium und insbesondere Cobalt, Rhodium und Ruthenium in beliebigen Oxidationsstufen verwendet.

Die Herstellung dieser Katalysatorsysteme ist technisch aufwendig und teuer. Dies gilt insbesondere, als daß die Katalysatorsysteme im Laufe ihrer Verwendung allmählich zersetzt werden und somit ausgeschleust und durch neuen Katalysator ersetzt werden müssen.

Verfahren zur Herstellung der Phosphorverbindungen und den entsprechenden Katalysatoren sind an sich bekannt, beispielsweise aus US 3,903,120, US 5,523,453, US 5,981,772, US 6,127,567, US 5,693,843, US 5,847,191, WO 01/14392, WO 99/13983 und WO 99/64155.

Zur Herstellung der in den Katalysatoren eingesetzten Phosphorverbindungen als Liganden kann man beispielsweise zunächst eine Dihalogenphosphor(III)verbindung mit einem Monoalkohol zu einem Diester umsetzen. Gewünschtenfalls kann diese Verbindung vor der weiteren Umsetzung nach bekannten Verfahren isoliert und/oder gereinigt werden, z. B. durch Destillation. Dieser Diester wird beispielsweise dann mit einem Diol zu den zweizähnigen Phosphonitliganden umgesetzt. Für den Fall, dass symmetrische Liganden erhalten werden sollen, können zwei Äquivalente des Diesters in einer einstufigen Reaktion mit einem Äquivalent des Diols umgesetzt werden. Ansonsten wird zunächst ein Äquivalent des Diesters mit einem Äquivalent des Diols umgesetzt und nach Bildung des Monokondensationsproduktes wird ein zweites Diol zugegeben und weiter zu der Phosphorverbindung umgesetzt.

Die in der Reaktion freigesetzte Säure kann mit einer der genannten Hilfsbase unter Ausbildung eines flüssigen Salzes abgefangen werden, so daß dies Synthese erheblich vereinfacht werden kann.

Organodiphosphonite der Formel III, sowie Katalysatorsysteme, die solche Organodiphosphonite enthalten, sind bekannt, beispielsweise aus WO 99/64155. Zur Herstellung solcher Organodiphosphonite der Formel III beschreibt WO 99/64155 die Umsetzung von R'PCl₂ mit einem Mol ROH und die nachfolgende Umsetzung des erhaltenen (RO)R'PCl mit einem halben mol, bezogen auf ein mol (RO)R'PCl, einer Verbindung HO-Z-OH bei einer Temperatur im Bereich von 40 bis etwa 200°C. Dabei sollte die Abspaltung des Halogenwasserstoffs in ersten Schritt bevorzugt rein thermisch erfolgen. Zudem sollen beide Schritte in Gegenwart einer Base durchgeführt werden können.

Die im Stand der Technik bekannten Verfahren, wie z.B. das aus der WO 99/64155 bekannte, zur Herstellung der genannten Phosphorverbindungen werden analog durchgeführt, mit dem Unterschied, daß eine Hilfsbase wie oben beschrieben eingesetzt wird und die freigesetzte Säure aus dem Reaktionsgemisch mittels der Hilfsbase abgetrennt wird, wobei wie oben die Hilfsbase ein Salz mit der Säure bildet, das bei Temperaturen flüssig ist, bei denen die Phosphorverbindung während der Abtrennung des Flüssigsalzes nicht signifikant zersetzt wird und das Salz der Hilfsbase mit der Phosphorverbindung oder der Lösung der Phosphorverbindung in einem geeigneten Lösungsmittel zwei nicht mischbare flüssige Phasen ausbildet.

Allgemein können die genannten Phosphorverbindungen beispielsweise wie folgt hergestellt werden:
Die Edukte werden in der gewünschten Stöchiometrie, gegebenenfalls in einem Lösungsmittel gelöst oder dispergiert, d.h. suspendiert oder emulgiert, miteinander vermischt. Dabei kann es sinnvoll sein, die Edukte in eine oder mehrere Zusammensetzungen, d.h. voneinander getrennte Ströme, aufzuteilen, so daß die Reaktion nicht vor der Vermischung stattfindet. Die Hilfsbase, die mit der Säure erfindungsgemäß ein flüssiges Salz bildet, kann einem oder mehreren dieser Ströme beigemischt werden oder getrennt von den Strömen als gesonderter Strom der Reaktion zugeführt werden. Es ist auch möglich, wenn auch weniger bevorzugt, die Hilfsbase erst nach der Reaktion zur Abtrennung der Säure zuzugeben.
Die Edukte oder die genannten Zusammensetzungen werden einem Reaktor zugeführt und unter Reaktionsbedingungen miteinander umgesetzt, die zur Reaktion der Edukte zum Produkt führen. Solche Reaktionsbedingungen sind abhängig von den eingesetzten Edukten und den gewünschten Produkten und in dem in dieser Schrift genannten Stand der Technik angegeben.

Die Reaktion kann kontinuierlich, halbkontinuierlich oder diskontinuierlich erfolgen. Die Temperatur reicht in der Regel von 40°C bis 200°C, der Druck ist erfindungsgemäß nicht wesentlich und kann Unter-, Über oder Normaldruck, beispielsweise von 10 mbar bis 10 bar, bevorzugt 20 mbar bis 5 bar, besonders bevorzugt 50 mbar bis 2 bar und insbesondere 100 mbar bis 1,5 bar betragen. Die Verweilzeit des Reaktionsgemischs im Reaktor kann von wenigen Sekunden bis mehreren Stunden betragen und ist von der Reaktionstemperatur und, in der Regel in geringerem Ausmaß, von dem angelegten Druck abhängig.

Bevorzugt wird die Verweilzeit bei einer kontinuierlichen Reaktionsführung bei einer für die Reaktion ausreichend hoher Temperatur kurz gewählt, d.h. von wenigen Sekunden bis ca. 2 Stunden, bevorzugt von 1 Sekunde bis 2 Stunden, besonders bevorzugt von 1 Sekunde bis 1 Stunde, ganz besonders bevorzugt von 1 Sekunde bis 30 Minuten, insbesondere von 1 Sekunde bis 15 Minuten und außergewöhnlich bevorzugt von 1 Sekunde bis 5 Minuten.

In einer besonders bevorzugten Ausführungsform wird die Herstellung der Phosphorverbindungen, bevorzugt solcher mit mehreren Phosphoratomen, besonders bevorzugt solcher mit 2 oder 3 und ganz besonders bevorzugt solcher mit 2 Phosphoratomen, aus den jeweiligen Edukten kontinuierlich bei einer Temperatur von 60°C bis 150°C, bevorzugt bei einer Temperatur oberhalb des Schmelzpunktes des Salzes der verwendeten Hilfsbase mit der freigesetzten Säure bis 130°C, bei einer Verweilzeit unter 1 Stunde, bevorzugt unter 30 Minuten, besonders bevorzugt unter 15 Minuten, ganz besonders bevorzugt von 1 Sekunde bis 5 Minuten, insbesondere von 1 Sekunde bis 1 Minute und außergewöhnlich bevorzugt von 1 bis 30 Sekunden durchgeführt.

Durch eine derartige Ausführungsform wird der Austausch von Substituenten an den Phosphoratomen zurückgedrängt und es ist so möglich, unter überwiegend kinetischer Kontrolle Verbindungen mit mehreren Phosphoratomen, wie beispielsweise Verbindungen der Formel (IX), und Phosphorverbindungen mit gemischten Substituenten, beispielsweise Verbindungen der Formel (VIII) mit unterschiedlichen Resten R⁷, R⁸ und/oder R⁹, herzustellen, ohne daß die Substituenten infolge Equilibrierung am Phosphoratom/an den Phosphoratomen ausgetauscht werden.

Während der Reaktion ist für eine gute Durchmischung zu sorgen, beispielsweise durch Rühren oder Umpumpen mit statischen Mischern oder Düsen.

Als Reaktoren können dem Fachmann an sich bekannte Apparate eingesetzt werden, beispielsweise ein oder mehrere kaskadierte Rühr- oder Rohrreaktoren mit innen- und/oder außenliegenden Heizungen und bevorzugt Reaktionsmischpumpen.

Der Reaktionsaustrag wird in einen Apparat geführt, in dem sich während der Reaktion entstandene Phasen voneinander trennen können, beispielsweise Phasenscheider oder Mixer-Settler-Apparaturen. In diesem Apparat wird bei einer Temperatur, bei der das Salz der Hilfsbase mit der Säure flüssig ist, eine Phasentrennung der Phase, die überwiegend ionische Flüssigkeit enthält, von der Phase, die überwiegend das gewünschte Reaktionsprodukt enthält, durchgeführt. Falls erforderlich kann Lösungsmittel hinzugegeben werden, um eine Phasentrennung zu beschleunigen.

Aus der Phase, die überwiegend ionische Flüssigkeit enthält, kann die Hilfsbase, wie oben beschrieben, wiedergewonnen werden.

Aus der Phase, die das gewünschte Reaktionsprodukt enthält, kann das Reaktionsprodukt mit an sich bekannten Methoden isoliert und/oder gereinigt werden, beispielsweise durch Destillation, Rektifikation, Extraktion, fraktionierter oder einfacher Kristallisation, Membrantrennverfahren, Chromatographie oder Kombinationen davon.

Bei dem in der Reaktion verwendeten Lösungsmittel kann es sich um die oben angeführten Lösungsmittel handeln.

Die in der Reaktion verwendete Hilfsbase wird in der Regel in, bezogen auf zu erwartende Menge Säure, stöchiometrischer Menge oder leichtem Überschuß eingesetzt, beispielsweise 100 bis 200 Mol% bezogen auf die zu erwartende Menge Säure, bevorzugt 100 bis 150 und besonders bevorzugt 105 bis 125 Mol%.

Die Edukte zur Herstellung der gewünschten Phosphorverbindungen sind dem Fachmann an sich bekannt oder leicht erschließbar und sind beispielsweise in dem in dieser Schrift genannten Stand der Technik angegeben, ebenso die stöchiometrischen Verhältnisse, um die Edukte miteinander zur Reaktion zu bringen.

Die Edukte werden möglichst als Flüssigkeiten oder Schmelzen eingesetzt, gegebenenfalls werden sie dazu in einem Lösungsmittel gelöst oder dispergiert. Selbstverständlich ist es aber auch möglich, die Edukte zumindest teilweise als Feststoffe einzusetzen.

Werden sie mit einem Lösungsmittel versetzt, so wird das Lösungsmittel in der Regel in einer derartigen Menge eingesetzt, daß das Gemisch flüssig ist, beispielsweise als Lösung oder Dispersion. Typische Konzentrationen der Edukte bezogen auf die Gesamtmenge der Lösung oder Dispersion sind 5 bis 95 Gew.-%, bevorzugt 10 bis 90 Gew.-%, besonders bevorzugt 25 bis 90 Gew.-% und ganz besonders bevorzugt 50 bis 90 Gew.-%.

Verbindungen (VIII) weisen die Formel

P (X¹R⁷) (X²R⁸) (X³R⁹) (VIII)

auf.

Unter Verbindung (VIII) wird eine einzelne Verbindung oder ein Gemisch verschiedener Verbindungen der vorgenannten Formel verstanden. Erfindungsgemäß sind X¹, X², X³ unabhängig voneinander Sauerstoff, Schwefel, NR¹⁰ oder Einzelbindung.

R¹⁰ steht darin für Wasserstoff oder einen organischen Rest mit 1 - 10 Kohlenstoffatomen, bevorzugt für Wasserstoff, Phenyl oder C₁ - C₄-Alkyl, worunter in dieser Schrift Methyl, Ethyl, iso-Propyl, n-Propyl, n-Butyl, iso-Butyl, sek-Butyl und *tert*-Butyl verstanden wird.

Falls alle der Gruppen X¹, X² und X³ für Einzelbindungen stehen, so stellt Verbindung (VIII) ein Phosphin der Formel P(R⁷ R⁸ R⁹) mit den für R⁷, R⁸ und R⁹ in dieser Beschreibung genannten Bedeutungen dar.

Falls zwei der Gruppen X¹, X² und X³ für Einzelbindungen stehen und eine für Sauerstoff, so stellt Verbindung (VIII) ein Phosphinit der Formel P(OR⁷)(R⁸)(R⁹) oder P(R⁷)(OR⁸)(R⁹) oder P(R⁷)(R⁸)(OR⁹) mit den für R⁷, R⁸ und R⁹ in dieser Beschreibung genannten Bedeutungen dar.

Falls eine der Gruppen X¹, X² und X³ für eine Einzelbindung steht und zwei für Sauerstoff, so stellt Verbindung (VIII) ein Phosphonit der Formel P(OR⁷)(OR⁸)(R⁹) oder P(R⁷)(OR⁸)(OR⁹) oder P(OR⁷)(R⁸)(OR⁹) mit den für R⁷, R⁸ und R⁹ in dieser Beschreibung genannten Bedeutungen dar.

In einer bevorzugten Ausführungsform sollten alle der Gruppen X¹, X² und X³ für Sauerstoff stehen, so daß Verbindung (VIII) vorteilhaft ein Phosphit der Formel P(OR⁷)(OR⁸)(OR⁹) mit den für R⁷, R⁸ und R⁹ in dieser Beschreibung genannten Bedeutungen darstellt.

R⁷, R⁸, R⁹ stehen unabhängig voneinander für gleiche oder unterschiedliche organische Reste.

Als R⁷, R⁸ und R⁹ kommen unabhängig voneinander Alkylreste, vorteilhaft mit 1 bis 10 C-Atomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, p-Fluor-Phenyl, 1-Naphthyl, 2-Naphthyl, oder Hydrocarbyl, vorteilhaft mit 1 bis 20 C-Atomen, wie 1,1'-Biphenol, 1,1'-Binaphthol in Betracht.

Die Gruppen R⁷, R⁸ und R⁹ können miteinander direkt, also nicht allein über das zentrale Phosphor-Atom, verbunden sein. Vorzugsweise sind die Gruppen R⁷, R⁸ und R⁹ nicht miteinander direkt verbunden.

In einer bevorzugten Ausführungsform kommen als Gruppen R⁷, R⁸ und R⁹ Reste ausgewählt aus der Gruppe bestehend aus Phenyl, o-Tolyl, m-Tolyl und p-Tolyl in Betracht.

In einer besonders bevorzugten Ausführungsform sollten dabei maximal zwei der Gruppen R⁷, R⁸ und R⁹ Phenyl-Gruppen sein.

In einer anderen bevorzugten Ausführungsform sollten dabei maximal zwei der Gruppen R⁷, R⁸ und R⁹ o-Tolyl-Gruppen sein.

Als besonders bevorzugte Verbindungen (VIII) können solche der Formel

(o-Tolyl-O-)_{w} (m-Tolyl-O-)ₓ (p-Tolyl-O-)_{y} (Phenyl-O-)_{z} P

mit w, x, y, z eine natürliche Zahl
mit w + x + y + z = 3 und
w, z kleiner gleich 2
eingesetzt werden, wie (p-Tolyl-O-)(Phenyl)₂P, (m-Tolyl-O-)(Phenyl)₂P, (o-Tolyl-O-)(Phenyl)₂P, (p-Tolyl-O-)₂(Phenyl)P, (m-Tolyl-O-)₂(Phenyl)P, (o-Tolyl-O-)₂(Phenyl)P, (m-Tolyl-O-)(p-Tolyl-O-)(Phenyl)P, (o-Tolyl-O-)(p-Tolyl-O-)(Phenyl)P, (o-Tolyl-O-)(m-Tolyl-O-)(Phenyl)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Tolyl-O-)P, (o-Tolyl-O-)₂(p-Tolyl-O-)P, (o-Tolyl-O-)(m-Tolyl-O-)(p-Tolyl-O-)P, (m-Tolyl-O-)₃P, (o-Tolyl-O-)(m-Tolyl-O-)₂P (o-Tolyl-O-)₂(m-Tolyl-O-)P oder Gemische solcher Verbindungen eingesetzt werden.

So können beispielsweise Gemische enthaltend (m-Tolyl-O-)₃P, (m-Tolyl-O-)₂(p-Tolyl-O-)P, (m-Tolyl-O-)(p-Tolyl-O-)₂P und (p-Tolyl-O-)₃P durch Umsetzung eines Gemisches enthaltend m-Kresol und p-Kresol, insbesondere im Molverhältnis 2:1, wie es bei der destillativen Aufarbeitung von Erdöl anfällt, mit einem Phosphortrihalogenid, wie Phosphortrichlorid, erhalten werden.

Solche Verbindungen (VIII) und deren Herstellung sind an sich bekannt.

Verbindungen (IX) weisen die Formel 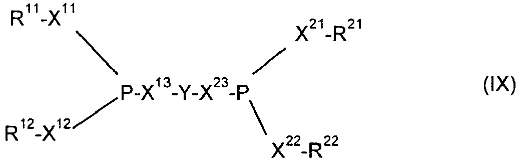 mit
X¹¹, X¹², X¹³ X²¹, X²², X²³ unabhängig voneinander Sauerstoff, Schwefel, NR¹⁰ oder Einzelbindung
R¹¹, R¹² unabhängig voneinander gleiche oder unterschiedliche, einzelne oder verbrückte organische Reste
R²¹, R²² unabhängig voneinander gleiche oder unterschiedliche, einzelne oder verbrückte organische Reste,
Y Brückengruppe
auf.

Unter Verbindung (IX) wird eine einzelne Verbindung oder ein Gemisch verschiedener Verbindungen der vorgenannten Formel verstanden.

In einer bevorzugten Ausführungsform können X¹¹, X¹², X¹³ X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so daß das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so daß das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so daß das mit X¹¹, X¹² und X¹³ umgebene Phosphoratom Zentralatom eines Phosphinits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so daß das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so daß das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so daß das mit X²¹, X²² und X²³ umgebene Phosphoratom Zentralatom eines Phosphits oder Phosphins, vorzugsweise eines Phosphins, sein kann.

Als Brückengruppe Y kommen vorteilhaft substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituierte, Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, inbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterschiedliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, inbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein.

Die Reste R²¹ und R²² können einzeln oder verbrückt sein.

Die Reste R¹¹, R¹², R²¹ und R²² können alle einzeln, zwei verbrückt und zwei einzeln oder alle vier verbrückt sein in der beschriebenen Art.

Auf die folgenden, besonders bevorzugten Ausführungsformen wird im Rahmen der vorliegenden Offenbarung im genannten Umfang ausdrücklich Bezug genommen:
In einer besonders bevorzugten Ausführungsform kommen die in US 3,773,809 genannten Verbindungen in Betracht, insbesondere die in Spalte 2, Zeile 23 bis Spalte 4, Zeile 14 und in den Beispielen beschriebenen.
In einer besonders bevorzugten Ausführungsform kommen die in US 6,127,567 genannten Verbindungen in Betracht, insbesondere die in Spalte 2, Zeile 23 bis Spalte 6, Zeile 35, in den Formeln I, II, III, IV, V, VI, VII, VIII und IX und in den Beispielen 1 bis 29 eingesetzten Verbindungen.
In einer besonders bevorzugten Ausführungsform kommen die in US 6,171,996 genannten Verbindungen in Betracht, insbesondere die in Spalte 2, Zeile 25 bis Spalte 6, Zeile 39, in den Formeln I, II, III, IV, V, VI, VII, VIII und IX und in den Beispielen 1 bis 29 eingesetzten Verbindungen.
In einer besonders bevorzugten Ausführungsform kommen die in US 6,380,421 genannten Verbindungen in Betracht, insbesondere die in Spalte 2, Zeile 58 bis Spalte 6, Zeile 63, in den Formeln I, II und III und in den Beispielen 1 bis 3 eingesetzten Verbindungen.
In einer besonders bevorzugten Ausführungsform kommen die in US 5,488,129 genannten Verbindungen in Betracht, insbesondere die in Spalte 3, Zeile 4 bis Spalte 4, Zeile 33, in der Formel I und in den Beispielen 1 bis 49 eingesetzten Verbindungen.
In einer besonders bevorzugten Ausführungsform kommen die in US 5,856,555 genannten Verbindungen in Betracht, insbesondere die in Spalte 2, Zeile 13 bis Spalte 5, Zeile 30, in den Formeln I und II und in den Beispielen 1 bis 4 eingesetzten Verbindungen.
In einer besonders bevorzugten Ausführungsform kommen die in WO 99/46044 genannten Verbindungen in Betracht, besonders die in Seite 3, Zeile 7 bis Seite 8, Zeile 27, und insbesondere die in den Formeln la bis Ig und in den Beispielen 1 bis 6 eingesetzten Verbindungen.
In einer besonders bevorzugten Ausführungsform kommen die in US 5,723,641 genannten Verbindungen der Formel I, II, III, IV und V in Betracht.
In einer besonders bevorzugten Ausführungsform kommen die in US 5,512,696 genannten Verbindungen der Formel I, II, III, IV, V, VI und VII, insbesondere die dort in den Beispielen 1 bis 31 eingesetzte Verbindungen, in Betracht.
In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzte Verbindungen, in Betracht.
In einer besonders bevorzugten Ausführungsform kommen die in US 5,512,695 genannten Verbindungen der Formel I, II, III, IV, V und VI, insbesondere die dort in den Beispielen 1 bis 6 eingesetzte Verbindungen, in Betracht.
In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzte Verbindungen, in Betracht.
In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzte Verbindungen, in Betracht.
In einer besonders bevorzugten Ausführungsform kommen die in US 5,959,135 genannten Verbindungen und dort in den Beispielen 1 bis 13 eingesetzte Verbindungen in Betracht.
In einer besonders bevorzugten Ausführungsform kommen die in US 5,847,191 genannten Verbindungen der Formel I, II und III in Betracht.
In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 12, 13, 14, 15, 16, 17, 18, 19, 20 und 21 dargestellten Verbindungen, in Betracht.
In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.
In einer besonders bevorzugten Ausführungsform kommen die in WO 98/27054 genannten Verbindungen in Betracht.
In einer besonders bevorzugten Ausführungsform kommen die in WO 99/13983 genannten Verbindungen in Betracht, besonders die auf Seite 5, Zeile 1 bis Seite 11, Zeile 45 und insbesondere die in den Formeln la bis Ih und den Beispielen 1 bis 24 genannten Verbindungen.
In einer besonders bevorzugten Ausführungsform kommen die in WO 99/64155 genannten Verbindungen in Betracht, besonders die auf Seite 4, Zeile 1 bis Seite 12, Zeile 7 und insbesondere die in den Formeln la bis Ic und den Beispielen 1 bis 4 genannten Verbindungen.
In einer besonders bevorzugten Ausführungsform kommen die in der deutschen Patentanmeldung DE-A 10038037 genannten Verbindungen in Betracht.
In einer besonders bevorzugten Ausführungsform kommen die in der deutschen Patentanmeldung DE-A 10046025 genannten Verbindungen in Betracht.
In einer besonders bevorzugten Ausführungsform kommen die in der deutschen Patentanmeldung DE-A 10156292 genannten Verbindungen in Betracht, insbesondere die auf Seite 1, Zeilen 6 bis 19 und von Seite 2, Zeile 21 bis Seite 2, Zeile 30 genannten Verbindungen.
In einer besonders bevorzugten Ausführungsform kommen die in der deutschen Patentanmeldung DE-A 10150281 genannten Verbindungen in Betracht, insbesondere auf Seite 1, Zeile 36 bis Seite 5, Zeile 45 genannten Verbindungen.
In einer besonders bevorzugten Ausführungsform kommen die in der deutschen Patentanmeldung DE-A 10150285 genannten Verbindungen in Betracht, insbesondere auf Seite 1, Zeile 35 bis Seite 5, Zeile 37 genannten Verbindungen.
In einer besonders bevorzugten Ausführungsform kommen die in der deutschen Patentanmeldung DE-A 10150286 genannten Verbindungen in Betracht, insbesondere auf Seite 1, Zeile 37 bis Seite 6, Zeile 15 genannten Verbindungen.
In einer besonders bevorzugten Ausführungsform kommen die in der deutschen Patentanmeldung DE-A 10148712 genannten Verbindungen in Betracht, insbesondere auf Seite 1, Zeilen 6 bis 29 und Seite 2, Zeile 15 bis Seite 4, Zeile 24 genannten Verbindungen.

In dieser Schrift verwendete ppm- und Prozentangaben beziehen sich, falls nicht anders angegeben, auf Gewichtsprozente und -ppm.

### Beispiele

### Vergleichsbeispiel 1 Herstellung von Diethoxyphenylphosphin (DEOPP)

In einem mit N₂ inertisierten 1000ml-Reaktor mit Impellerrührer wurden 101,4 g Ethanol, 543 g Xylol und 232,7 g Triethylamin vorgelegt und auf 50°C aufgeheizt. In diese Mischung tropfte man innerhalb von 40 Minuten 181,5 g 98,6 %iges Dichlorphenylphosphin, worauf sich eine farblose, gut rührbare Suspension bildete. Durch Kühlen wurde die Reaktionstemperatur auf 50°C gehalten. Nach vollständiger Zugabe des Dichlorphenylphosphins wurde der Ansatz noch 60 Minuten bei 75-80°C nachgerührt und anschließend das ausgefallene Hydrochlorid abgesaugt und mit kaltem Xylol gewaschen. Filtrat und Waschxylol wurden vereinigt (insges. 859,9 g) und mittels GC mit internem Standard untersucht. Die xylolische Lösung enthielt 11,8 % Diethoxyphenylphosphin, was einer Ausbeute von 51 % entspricht.

### Vergleichsbeispiel 2 Herstellung von Diethoxyphenylphosphin (DEOPP)

In einem mit N₂ inertisierten 1000ml-Reaktor mit Impellerrührer wurden 90,9 g Ethanol und 382,2 g Tributylamin vorgelegt und auf 70°C aufgeheizt. In diese Mischung tropfte man innerhalb von 40 Minuten 162,7 g 98,6 %iges Dichlorphenylphosphin, worauf sich eine farblose Lösung bildete, die in der Wärme flüssig war und nach Abkühlen auf Ramutemperatur zu einem farblosen, kristallinem Feststoff erstarrte. Durch Kühlen wurde die Reaktionstemperatur auf 50°C gehalten. Nach vollständiger Zugabe des Dichlorphenylphosphins wurde der Ansatz noch 60 Minuten bei 75-80°C nachgerührt. Die 625,8 g Reaktionsaustrag enthielten laut GC mit internem Standard 23,7 % Diethoxyphenylphosphin, was einer Ausbeute von 82,7 % entspricht.

### Beispiel 1: Herstellung von Diethoxyphenylphosphin (DEOPP)

In einem mit N₂ inertisierten 1000ml-Reaktor mit Schrägblattrührer wurden 188,9 g (2,3 mol) 1-Methylimidazol und 101,4 g (2,2 mol) Ethanol vorgelegt. Innerhalb 90 min wurden nun 181,5 g (1,0 mol) 98,6 %iges Dichlorphenylphosphin dosiert. Dabei wurde zunächst eine Erwärmung auf 60°C zugelassen (Dauer: 6 min) und anschließend durch Kühlen die Temperatur bei der weiteren Zugabe auf 60°C gehalten. Nach Zulaufende war der Ansatz noch flüssig, kristallisierte aber in der Nachrührzeit von 45 min aus. Nach Aufheizen auf 80°C war das Reaktionsgemisch wieder restlos flüssig. Nach weiterem einstündigen Rühren wurde der Rührer ausgeschaltet. Es bildeten sich rasch zwei gut getrennte Phasen aus. Nach Phasentrennung bei 80 °C wurden 199,4 g einer klaren, farblosen Oberphase (DEOPP-Gehalt nach GC: 96,1 %; Gehalt an 1-Methylimidazol 1,7 %) und 266,4 g Unterphase ("ionische Flüssigkeit") erhalten.

Die Oberphase wurde im Vakuum über eine 40 cm Kolonne mit 5 mm Raschigringen destilliert. Dabei erhielt man 15,8 g eines klaren, farblosen Vorlaufs (GC: 76,9 % DEOPP-Gehalt) und 177,5 g eines farblosen Hauptlaufs (GC: 99,4 % DEOPP). Im Kolben blieben nur 4,3 g Sumpf zurück, der nach GC noch 11,1 % DEOPP enthielt. Die DEOPP-Ausbeute nach Destillation betrug 95,9 %.

### Beispiel 2

### Herstellung von Triethylphosphit (TEP)

In einem mit N₂ inertisierten 1000ml-Reaktor mit Schrägblattrührer wurden 425 g 1-Methylimidazol und 228,1 g Ethanol vorgelegt. Innerhalb 190 min wurden nun unter Eiskühlung bei 23-33°C Innentemperatur 206 g Phosphortrichlorid zugetropft. Die Reaktion verlief exotherm, so daß gekühlt wurden mußte, um diese Temperatur zu halten. Nach etwa der halben Zugabe wurde der Reaktionsansatz trübe, wobei zwei flüssige Phasen erhalten wurden. Die obere bestand laut GC aus 90,0 % Triethylphosphit, die untere aus dem Hydrochlorid des 1-Methylimidazol. Vor der Phasentrennung wurde auf 78°C aufgeheizt. Es wurden 231,4 g einer farblosen Oberphase und 611,9 g einer klaren Unterphase erhalten. Die Oberphase wurde im Vakuum über eine 30cm Glaskolonne mit einer Sulzer DX Packung destilliert. Es wurden 177 g Triethylphosphit mit einer Reinheit von 99 % erhalten. Im Vor- und Nachlauf waren weitere 28,3 g Triethylphosphit enthalten. Die Gesamtausbeute betrug 82,4 %.

### Beispiel 3

### Herstellung von Diethoxyphenylphosphin (DEOPP)

In einem 250 ml Glaskolben mit Teflonblattrührer wurden 85,7 g 2-Methylpyridin und 40,5 g Ethanol vorgelegt. Unter Kühlung wurden innerhalb 25 min 71,6 g Dichlorphenylphosphin (98,6 %ig) zugetropft, so daß die Innentemperatur bei 20-29°C blieb. Während der Zugabe fiel das Hydrochlorid des 2-Methylpyridins aus. Nach vollständiger Zugabe wurde der Ansatz aufgeheizt, wobei das Hydrochlorid ab etwa 70°C zu schmelzen begann. Es bildeten sich zwei klare, scharf getrennte flüssige Phasen aus, wobei 75,5 g Oberphase und 115,8 g Unterphase erhalten wurden. Die Oberphase enthielt 81,6 % DEOPP, so daß die Ausbeute 77,7 % betrug.

Wurde die Unterphase mit wäßriger Natronlauge neutralisiert, so bildete sich erneut ein Zweiphasensystem aus, wobei die untere aus einer wäßrigen Kochsalzlösung und die obere aus dem freigesetzten 2-Methylpyridin bestand, das auf diese Weise durch eine einfache Flüssig-flüssig-Phasentrennung zurückgeführt werden konnte.

### Beispiel 4

### Herstellung von Ethoxydiphenylphosphin (EODPP)

In einem mit N₂ inertisierten 1000ml-Reaktor mit Schrägblattrührer wurden 141,7 g 1-Methylimidazol und 76,0 g Ethanol vorgelegt, in das 315,8 g Chlordiphenylphosphin innerhalb 30 min getropft wurden, wobei sich zwei flüssige Phasen ausbildeten. Die Innentemperatur wurde unterhalb von 65°C gehalten. Nach vollständiger Zugabe heizte man auf 75°C auf, rührte 45 min und trennte die Phasen, wobei 194,3 g Unter- und 332,8 g Oberphase erhalten wurden. Die Oberphase enthielt It. GC zu 96,6 % das Produkt EODPP. Zur weiteren Aufreinigung wurde die Oberphase im Vakuum über eine Glaskolonne mit Raschigringen destilliert, wobei 292,5 g 99,4 %iges EODPP erhalten wurden. Zusammen mit dem EODPP im Vorlauf betrug die Gesamtausbeute 92,2 %.

Die Unterphase, die aus dem flüssigen Hydrochlorid des 1-Methylimidazols bestand, wurde mit 244,1 g 25 %iger Natronlauge versetzt. Um das ausgefallene Kochsalz vollständig zu lösen, wurden weitere 94,3 g Wasser zugegeben, bis eine klare Lösung erhalten wurde. Nach Zugabe von 450 g n-Propanol fiel erneut Kochsalz aus, das nach weiterer Zugabe von 69,8 g Wasser wieder in Lösung gebracht wurde. Man erhielt zwei flüssige Phasen, wobei die 739,3 g Oberphase 19,99 % Wasser und 16.7 % 1-Methylimidazol enthielt. Das sind 94,9 % der bei der Synthese eingesetzten Menge an 1-Methylimidazol. Die 304,2 g Unterphase enthielt neben dem Kochsalz 70,6 % Wasser und 2,2 % 1-Methylimidazol. Durch erneute Extraktion mit n-Propanol konnte der Gehalt an 1-Methylimidazol in der wässrigen Phase auf 0,4 % gesenkt werden. 1-Methylimidazol konnte nun wiedergewonnen werden, indem das Gemisch aus Propanol und Wasser aus der Oberphase der ersten Extraktion abdestilliert wurde.

### Beispiel 5

### Kontinuierliche Herstellung von Ethoxydiphenylphosphin (EODPP)

In einen mit Stickstoff inertisierten Reaktor mit dreistufigem Schrägblattrührer wurden kontinuierlich bei 80°C folgende Einsatzstoffe zugefördert: 1) Mischung aus 110,7 g Ethanol und 205,8 g 1-Methylimidazol 2) Chlordiphenylphosphin (99,4 %ig). Strom 1) wurde mit 330 ml/h und Strom 2) mit 380 ml/h zugegeben. Beide Zuläufe erfolgten getaucht. Der Reaktor war mit einem Überlauf ausgestattet, aus dem kontinuierlich Reaktionsgemisch ablaufen konnte. Das Reaktorvolumen bis zum Überlauf betrug 710 ml. Die Reaktionstemperatur wurde auf 80°C gehalten. Um das System ins Gleichgewicht zu bringen, wurde der Austrag der ersten 4 h verworfen. Anschließend wurde der Austrag über eine Zeitdauer von 1 h gesammelt und bilanziert. Der Austrag bestand aus zwei flüssigen Phasen. Innerhalb von einer Stunde wurden 497,2 g Ober- und 280,8 g Unterphase gesammelt. Die Oberphase bestand zu 96,8 % aus EODPP. Die Oberphase wurde anschließend im Vakuum über eine mit Raschigringen gefüllte Kolonne destilliert, wobei 438,2 g 99,74 %iges EODPP erhalten wurden. Zusammen mit dem EODPP im Vorlauf betrug die Gesamtausbeute 96,7 %.

### Beispiel 6

### Kontinuierliche Herstellung von Ethoxydiphenylphosphin (EODPP)

In einer Reaktionsmischpumpe wurden kontinuierlich folgende Zulaufströme gemischt: 1) Mischung aus 159,2 g 1-Methylimidazol und 85,4 g Ethanol 2) 372,8 g Chlordiphenylphosphin (99,1 %ig). Vom Strom 1) wurden 1257 g/h zugegeben, vom Strom 2) 1928 g/h. Das Volumen der Mischkammer betrug 3,3 ml. Der Kopf der Reaktionsmischpumpe wurde auf 120°C thermostatisiert. Das System wurde 5 min ins Gleichgewicht gebracht. Anschließend wurde der Austrag über 11 min gesammelt, um zu bilanzieren. Während der Bilanzfahrt wurde die Menge an Einsatzstoffen durch Wiegen der Vorlagen bestimmt. Es wurden 372,8 g Chlordiphenylphosphin zugegeben. Der Austrag bestand aus zwei flüssigen Phasen. In den 11 min wurden 392,2 g Ober- und 218,3 g Unterphase gesammelt. Die Oberphase bestand zu 96,5 % aus EODPP, so daß die gaschromatographisch bestimmte Ausbeute 98,2 % betrug. Die Verweilzeit der Reaktanden in der Mischkammer betrug 4 s. Dadurch ergab sich eine Raum-Zeit-Ausbeute von 0,69•10⁶kgm⁻³h¹.

### Beispiel 7

### Kontinuierliche Herstellung von Ethoxydiphenylphosphin (EODPP)

In einer Reaktionsmischpumpe wurden kontinuierlich folgende Zulaufströme gemischt: 1) Mischung aus 156,7 g 1-Methylimidazol und 84,1 g Ethanol 2) 370,0 g Chlordiphenylphosphin (99,1 %ig). Vom Strom 1) wurden 167,5 g/h zugegeben, vom Strom 2) 257,4 g/h. Das Volumen der Mischkammer betrug 3,3 ml. Der Kopf der Reaktionsmischpumpe wurde auf 80°C thermostatisiert. Das System wurde 60 min ins Gleichgewicht gebracht. Anschließend wurde der Austrag über 87 min gesammelt, um zu bilanzieren. Während der Bilanzfahrt wurde die Menge an Einsatzstoffen durch Wiegen der Vorlagen bestimmt. Es wurden 370,0 g Chlordiphenylphosphin zugegeben. DerAustrag bestand aus zwei flüssigen Phasen. In den 87 min wurden 389,3 g Ober- und 219,2 g Unterphase gesammelt. Die Oberphase bestand zu 96,8 % aus EODPP, so daß die gaschromatographisch bestimmte Ausbeute 98,5 % betrug. Die Verweilzeit der Reaktanden in der Mischkammer betrug 30 s.

### Beispiel 8

### Kontinuierliche Herstellung von Diethoxyphenylphosphin (DEOPP)

In einer Reaktionsmischpumpe wurden kontinuierlich folgende Zulaufströme gemischt: 1) Mischung aus 237,1 g 1-Methylimidazol und 127,2 g Ethanol 2) 225,8 g Dichlorphenylphosphin. Vom Strom 1) wurden 385,6 g/h zugegeben, vom Strom 2) 239,0 g/h. Das Volumen der Mischkammer betrug 3,3 ml. Der Kopf der Reaktionsmischpumpe wurde auf 80°C thermostatisiert. Das System wurde 30 min ins Gleichgewicht gebracht. Anschließend wurde der Austrag über 58 min gesammelt, um zu bilanzieren. Während der Bilanzfahrt wurde die Menge an Einsatzstoffen durch Wiegen der Vorlagen bestimmt. Es wurden 225,8 g Dichlorphenylphosphin zugegeben. Der Austrag bestand aus zwei flüssigen Phasen. In den 58 min wurden 249,0 g Ober- und 335,6 g Unterphase gesammelt. Die Oberphase bestand zu 95,4 % aus DEOPP, so daß die gaschromatographisch bestimmte Ausbeute 95,5 % betrug. Die Verweilzeit der Reaktanden in der Mischkammer betrug 20 s.

### Beispiel 9

### Kontinuierliche Herstellung von Diethoxyphenylphosphin (DEOPP)

In einer Reaktionsmischpumpe wurden kontinuierlich folgende Zulaufströme gemischt: 1) Mischung aus 212,0 g 1-Methylimidazol und 113,7 g Ethanol 2) 201,7 g Dichlorphenylphosphin 3) rückgeführte Oberphase des Reaktionsaustrages. Vom Strom 1) wurden 1543,5 g/h zugegeben, vom Strom 2) 955,9 g/h, vom Strom 3) 2377 ml/h. Das Volumen der Mischkammer betrug 3,3 ml. Der Kopf der Reaktionsmischpumpe wurde auf 80°C thermostatisiert. Das System wurde 5 min ins Gleichgewicht gebracht. Anschließend wurde der Austrag über 12 min gesammelt, um zu bilanzieren. Während der Bilanzfahrt wurde die Menge an Einsatzstoffen durch Wiegen der Vorlagen bestimmt. Es wurden 201,7 g Dichlorphenylphosphin zugegeben. Der Austrag bestand aus zwei flüssigen Phasen, die in einem kontinuierlich betriebenen Phasenscheider getrennt wurden. Ein Teil der Oberphase wurde in den Prozeß zurückgeführt. In 12 min Bilanzfahrt wurden 227,0 g Ober- und 300,6 g Unterphase gesammelt. Die Oberphase bestand zu 95,2 % aus DEOPP, so daß die Ausbeute 97,2 % betrug. Die Verweilzeit der Reaktanden in der Mischkammer betrug 2,5 s. Dadurch ergibt sich eine Raum-Zeit-Ausbeute von 0,36•10⁶kgm⁻³h⁻¹.

### Beispiel 10

### Regenerierung von 1-Methylimidazol Hydrochlorid

Analog zu Beispiel 1 wurden aus 181,5 g Dichlorphenylphosphin, 101,4 g Ethanol und 189 g 1-Methylimidazol DEOPP hergestellt, wobei 202,2 g Oberphase mit einem DEOPP-Gehalt von 93,9 % und 265,5 g Unterphase anfallen. Die Oberphase enthält zudem noch 3,7 g 1-Methylimidazol. Die Unterphase wurde mit 169,6 g Paraffinöl gemischt. In diese Mischung wurde nun 168 g 50 %ige Natronlauge getropft, wobei eine gut rührbare Suspension erhalten wurde. Nach der Zugabe von 12,9 g Xylol und 78,4 g rückgeführten Xylols aus einem vorherigen Versuch, das noch 3,8 g 1-Methylimidazol enthielt, wurde mit Hilfe von Xylol bei Wasser ausgekreist. Insgesamt wurden 132,7 g Wasser ausgekreist. Wenn kein Wasser mehr abgeschieden wurde, destillierte man bei 30-85 mbar und 57 - 90°C Kopftemperatur das Xylol aus dem Reaktionsgemisch über eine 30 cm Füllkörperkolonne, wobei 88,4 g Destillat erhalten wurden, das 21,8 g 1-Methylimidazol enthielt. Das Destillat wurde im nächsten Vesuch als rückgeführtes Xylol wieder eingesetzt, so daß darin enthaltenes 1-Methylimidazol immer wieder in den Prozeß zurückgeführt wurde. Nach der Xyloldestillation wurde bei 30 mbar und 90°C Kopftemperatur das 1-Methylimidazol abdestilliert. Es wurden 164,0 g 1-Methylimidazol wiedergewonnen, das einen Gehalt von 99,7 % aufweist. Der Wassergehalt des destillierten 1-Methylimidazols betrug 0,06 %.

Der Destillationssumpf wurde nun mit 350 g Wasser versetzt, um das im Weißöl suspendierte Kochsalz zu lösen. Es bildeten sich zwei Phasen aus. Die 475,7 g Unterphase enthielten das Kochsalz und 0,3 % (1,4 g) 1-Methylimidazol. Die 161,1 g Oberphase bestanden aus dem Weißöl, das als inertes Suspendierhilfsmittel ebenfalls wieder in den Prozeß zurückgeführt wurde. Von den insgesamt eingesetzten 192,8 g 1-Methylimidazol (189,0 g frisch und 3,8 g im rückgeführten Xylol) wurden 164,0 g als Reinstoff wiedergewonnen. Weitere 21,8 g befanden sich im abdestillierten Xylol, das in den Prozeß zurückgeführt wurde und damit erhalten bleibt. Insgesamt konnten somit 185,8 g (96 %) des 1-Methylimidazols zurückgeführt wurden.

### Beispiel 11

51 g Essigsäure wurden in 120,8 g Cyclohexan gelöst. Um die Säure wieder zu entfernen, wurden nun 69,80 g 1-Methylimidazol in die Lösung gegeben, worauf sich ein Zweiphasengemisch, bestehend aus 119,4 g Oberphase (Cyclohexan) und 122,5 g Unterphase (Ionische Flüssigkeit = 1-Methylimidazoliumacetat) bildete. Während der Zugabe von 1-Methylimidazol stieg die Temperatur aufgrund der Salzbildung bis auf 40°C an. Durch Kühlen mit einem Eisbad wurde die Temperatur bei der weiteren Zugabe auf 40°C gehalten. Nach Abkühlen konnte die Essigsäure nahezu vollständig in Form der gebildeten Ionischen Flüssigkeit, die mit Cyclohexan nicht mischbar ist, von dem Lösemittel über eine Flüssig-Flüssig-Phasentrennung abgetrennt werden.

### Beispiel 12

### Kontinuierliche Herstellung des folgenden Chelatphosphonits:

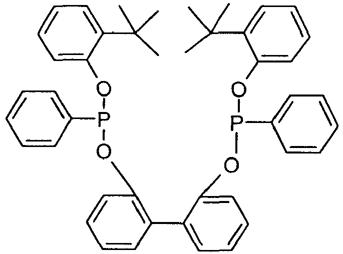

In einer Reaktionsmischpumpe wurden kontinuierlich folgende Zulaufströme gemischt:
1) Zusammensetzung: Mischung aus 11,9 g 1-Methylimidazol, 11,8 g o-Biphenol und 35,1 g Toluol und
2) Zusammensetzung: Mischung aus 38,4 g (2-tert-butylphenoxy)-chlorphenylphosphin und 153,5 g Toluol.

Vom Strom 1) wurden 681 ml/h, vom Strom 2) 2373 ml/h dosiert. Das Volumen der Mischkammer betrug 3,3 ml. Der Kopf der Reaktionsmischpumpe wurde auf 120°C thermostatisiert. Das System wurde 3 min ins Gleichgewicht gebracht. Anschließend wurde der Austrag über 7 min gesammelt, um zu bilanzieren. Die Temperatur des Reaktionsmediums am Ausgang der Reaktionsmischpumpe betrug 100°C. Der Austrag bestand aus zwei flüssigen Phasen, die in einem Gefäß gesammelt und anschließend getrennt wurden. In 7 min Bilanzfahrt wurden 233,9 g Ober- und 14,0 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Selektivität des erwünschten Chelatphosphonits gegenüber den unerwünschten monodentaten Phosphoniten wurde mit Hilfe von 31 P-NMR-Spektren ermittelt. Sie betrug 93,8 % zugunsten des Chelatphosphonits. Der Umsatz war vollständig.

### Beispiel 13

Die Synthese des Chelatphosphonits aus Beispiel 12 wurde durchgeführt wie unter Beispiel 12 beschrieben. Es wurden verschiedene Parameter variiert. Der Kopf der Reaktionsmischpumpe wurde so thermostatisiert, daß die in der Tabelle angegebenen Endtemperaturen des Reaktionsgemisches am Ausgang der Pumpe erhalten werden konnten. Die Ergebnisse sind in folgender Tabelle zusammengefaßt.

| Zusammenset zung Strom 1 | Zusammenset zung Strom 2 | Zulauf Strom 1 | Zulauf Strom 2 | Temperatur Reaktorausgang | Selektivität Chelatphosphonit in Bezug auf monodentate Phosphonite |
|---|---|---|---|---|---|
| 33,3 g MIA | 106,0 g TBCP | 1603 ml/h | 1367 ml/h | 105,5°C | 96,6% |
| 32,8 g BP | 45,4 g Tol | | | | |
| 98,0 g Tol | | | | | |
| 37,3 g MIA | 118,7 g TBCP | 1603 ml/h | 1367 ml/h | 90,5°C | 97,3 % |
| 36,7 g BP | 50,9 g Tol | | | | |
| 109,7 g Tol | | | | | |
| 41,3 g MIA | 130,9 g TBCP | 1603 ml/h | 1367 ml/h | 76,8°C | 98,6 % |
| 40,7 g BP | 56,1 g Tol | | | | |
| 121,6 g Tol | | | | | |
| 41,3 g MIA | 130,9 g TBCP | 1603 ml/h | 1367 ml/h | 76,8 °C | 98,6 % |
| 40,7 g BP | 56,1 g Tol | | | | |
| 121,6 g Tol | | | | | |
| 21,2 g MIA | 71,2 g TBCP | 1270 ml/h | 1156 ml/h | 76,3 °C | 99,3 % |
| 20,9 g BP | 30,5 g Tol | | | | |
| 62,5 g Tol | | | | | |

| | | | | | |
|---|---|---|---|---|---|
| MIA = 1-Methylimidazol BP = o-Biphenol Tol = Toluol TBCP = (2-tert-butylphenoxy)-chlorphenylphosphin | | | | | |

Der Umsatz war bei allen Varianten vollständig.

### Beispiel 14

### Kontinuierliche Herstellung des folgenden Chelatphosphonits:

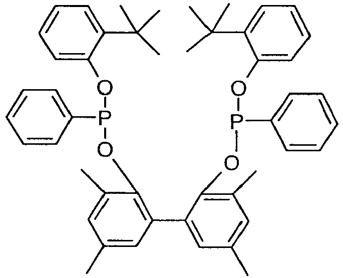

In einer Reaktionsmischpumpe wurden kontinuierlich folgende Zulaufströme gemischt:
1) Zusammensetzung: Mischung aus 28,0 g 1-Methylimidazol, 36,1 g 2,2',4,4'-Tetramethyl-o-Biphenol und 116,4 g Toluol und
2) Zusammensetzung: Mischung aus 88,4 g (2-tert-butylphenoxy)-chlorphenylphosphin und 37,9 g Toluol.

Vom Strom 1) wurden 1817 ml/h, vom Strom 2) 1153 ml/h dosiert. Das Volumen der Mischkammer betrug 3,3 ml. Das System wurde 2 min ins Gleichgewicht gebracht. Anschließend wurde der Austrag über 5 min gesammelt, um zu bilanzieren. Die Temperatur des Reaktionsmediums am Ausgang der Reaktionsmischpumpe betrug 76,3°C. Der Austrag bestand aus zwei flüssigen Phasen, die in einem Gefäß gesammelt und anschließend getrennt wurden. In 5 min Bilanzfahrt wurden 264,3 g Ober- und 40,1 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Selektivität des erwünschten Chelatphosphonits gegenüber den unerwünschten monodentaten Phosphoniten wurde mit Hilfe von 31 P-NMR-Spektren ermittelt. Sie betrug 95,6 % zugunsten des Chelatphosphonits. Der Umsatz war vollständig. Die Unterphase (ionische Flüssigkeit) enthielt nur ca. 300 ppm an phosphorhaltigen Nebenkomponenten.

### Beispiel 15

### Kontinuierliche Herstellung des folgenden Chelatphosphonits:

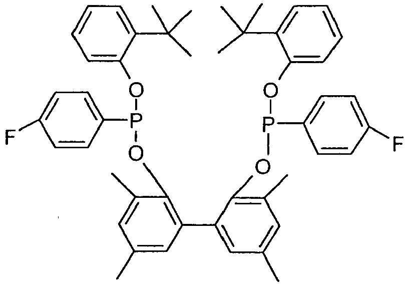

In einer Reaktionsmischpumpe wurden kontinuierlich folgende Zulaufströme gemischt:
1) Zusammensetzung: Mischung aus 188,9 g 1-Methylimidazol, 249,1 g 2,2',4,4'-Tetramethyl-o-Biphenol und 807,4 g Toluol und
2) Zusammensetzung: Mischung aus 664,7 g (2-tert-butylphenoxy)-p-fluorphenyl-chlorphosphin und 284,9 g Toluol.

Vom Strom 1) wurden 1781 ml/h, vom Strom 2) 1189 ml/h dosiert. Das Volumen der Mischkammer betrug 3,3 ml. Das System wurde 2 min ins Gleichgewicht gebracht. Anschließend wurde der Austrag über 275 min gesammelt, um zu bilanzieren. Die Temperatur des Reaktionsmediums am Ausgang der Reaktionsmischpumpe betrug 69,8°C. Der Austrag bestand aus zwei flüssigen Phasen, die in einem Gefäß gesammelt und anschließend getrennt wurden. In 275 min Bilanzfahrt wurden 799,6 g Ober- und 98,9 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 302,9 g (93,4 % d.Th).

### Beispiel 16

### Kontinuierliche Herstellung des folgenden Chelatphosphonits:

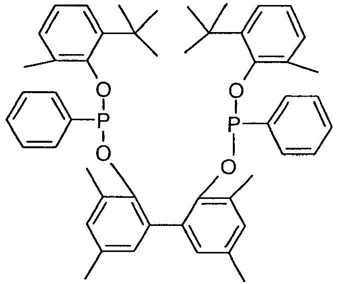

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 696,1 g (2-tert-Butyl-6-methylphenoxy)-chlorphenylphosphin und 298,3 g Toluol.

Vom Strom 1) wurden 1730 ml/h, vom Strom 2) 1238 ml/h dosiert. Das Volumen der Mischkammer betrug 3,3 ml. Das System wurde 2 min ins Gleichgewicht gebracht. Anschließend wurde der Austrag über 275 min gesammelt, um zu bilanzieren. Die Temperatur des Reaktionsmediums am Ausgang der Reaktionsmischpumpe betrug 69,5°C. Der Austrag bestand aus zwei flüssigen Phasen, die in einem Gefäß gesammelt und anschließend getrennt wurden. In 275 min Bilanzfahrt wurden 798,1 g Ober- und 93,3 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 298,3 g (95,2 % d.Th).

### Beispiel 17

### Kontinuierliche Herstellung des folgenden Chelatphosphits:

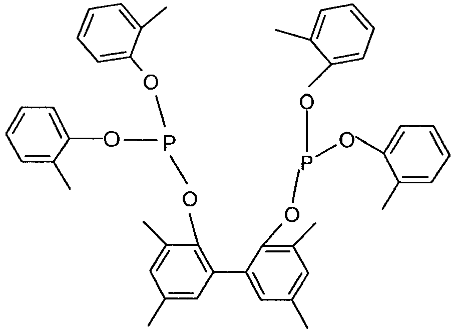

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 660,5 g (Di-o-kresyl)-chlorphosphin und 283,1 g Toluol. Vom Strom 1) wurden 1793 ml/h, vom Strom 2) 1176 ml/h dosiert. Das Volumen der Mischkammer betrug 3,3 ml. Das System wurde 2 min ins Gleichgewicht gebracht. Anschließend wurde der Austrag über 160 min gesammelt, um zu bilanzieren. Die Temperatur des Reaktionsmediums am Ausgang der Reaktionsmischpumpe betrug 70,1°C. Der Austrag bestand aus zwei flüssigen Phasen, die in einem Gefäß gesammelt und anschließend getrennt wurden. In 160 min Bilanzfahrt wurden 470,8 g Ober- und 60,8 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 166,6 g (93,0 % d.Th).

### Beispiel 18

### Kontinuierliche Herstellung des folgenden Chelatphosphinits:

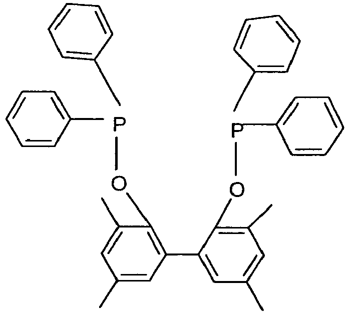

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 445,8 g Diphenylchlorphosphin und 191,1 g Toluol. Vom Strom 1) wurden 1991 ml/h, vom Strom 2) 906 ml/h dosiert. Das Volumen der Mischkammer betrug 3,3 ml. Das System wurde 2 min ins Gleichgewicht gebracht. Anschließend wurde der Austrag über 218 min gesammelt, um zu bilanzieren. Die Temperatur des Reaktionsmediums am Ausgang der Reaktionsmischpumpe betrug 70,1°C. Der Austrag bestand aus zwei flüssigen Phasen, die in einem Gefäß gesammelt und anschließend getrennt wurden. In 218 min

Bilanzfahrt wurden 641,8 g Ober- und 93 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 152,3 g (67,4 % d.Th).

### Beispiel 19

### Kontinuierliche Herstellung des folgenden Chelatphosphonits:

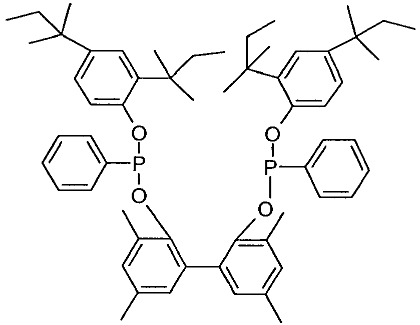

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 828,1 g (2,4-Di-isoamylphenoxy)- chlorphenylphosphin und 354,9 g Toluol.

Vom Strom 1) wurden 1532 ml/h, vom Strom 2) 1395 ml/h dosiert. Das Volumen der Mischkammer betrug 3,3 ml. Das System wurde 2 min ins Gleichgewicht gebracht. Anschließend wurde der Austrag über 275 min gesammelt, um zu bilanzieren. Die Temperatur des Reaktionsmediums am Ausgang der Reaktionsmischpumpe betrug 69°C. Der Austrag bestand aus zwei flüssigen Phasen, die in einem Gefäß gesammelt und anschließend getrennt wurden. In 275 min Bilanzfahrt wurden 787,9 g Ober- und 85,3 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 304 g (89,6 % d.Th).

### Beispiel 20

### Kontinuierliche Herstellung des folgenden Chelatphosphonits:

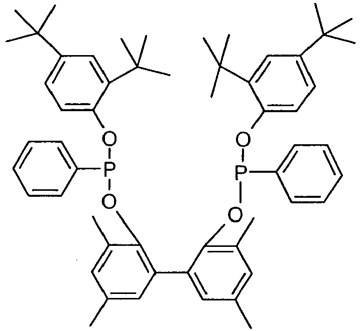

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 738,3 g (2,4-Di-tert-butylphenoxy)-chlorphenylphosphin und 316,4 g Toluol.

Vom Strom 1) wurden 1664 ml/h, vom Strom 2) 1308 ml/h dosiert. Das Volumen der Mischkammer betrug 3,3 ml. Das System wurde 2 min ins Gleichgewicht gebracht. Anschließend wurde der Austrag über 233 min gesammelt, um zu bilanzieren. Die Temperatur des Reaktionsmediums am Ausgang der Reaktionsmischpumpe betrug 75,8°C. Der Austrag bestand aus zwei flüssigen Phasen, die in einem Gefäß gesammelt und anschließend getrennt wurden. In 233 min Bilanzfahrt wurden 663,9 g Ober- und 79,8 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 267 g (94,7 % d.Th).

### Beispiel 21

In einem 1I-Kolben mit thermostatiertem Doppelmantel, mechanischer Rührung, Thermometer und Rückflußkühler wurden unter Argonatmosphäre ein Gemisch aus 1,7 mol PCl₃ und 0,6 mol AlCl₃ (98% Reinhheit) bei 73 °C vorgelegt. Anschließend wurden innerhalb von 30 min 0,4 mol Fluorbenzol hinzugegeben, wobei ein leichter Argonstrom durch den Reaktionskolben geleitet wurde. Das Reaktionsgemisch wurde 3 Std gerührt, auf 60 °C abgekühlt und 0,62 mol N-Methylimidazol innerhalb von 45 min langsam zugegeben. Die Reaktion war exotherm und es entstanden Nebel. Anschließend wurde noch 30 min bei 60 °C weitergerührt. Bei Abstellen der Rührung trennten sich 2 Phasen. Die untere Phase wurde abgetrennt und die obere Phase bei 60 °C zweimal mit je 80 ml PCl₃ extrahiert.

Die Unterphase und die vereinigten PCl₃-Extrakte wurden destilliert, wobei man 55 g p-Fluorphenyl-dichlorphosphin in einer Ausbeute von 70 % d. Th. und einer Reinheit von 96 % (bestimmt per ³¹P-NMR) erhielt.

### Beispiele 22 - 27

Es wurde verfahren wie in Beispiel 21 beschrieben, jedoch wurden die in der Tabelle angegebenen Verhältnisse an Fluorbenzol, AlCl₃, PCl₃ und N-Methylimidazol verwendete.

| Bsp. | Molverhältnis AlCl₃: Fluorbenzol | Molverhältnis N-Methylimidazol: AlCl₃ | Reaktionsdauer [h] | Ausbeu te [%] | Reinheit [%] |
|---|---|---|---|---|---|
| 21 | 1,5 | 1 | 3 | 70 | 96 |
| 22 | 1,5 | 1 | 6 | 65 | 96 |
| 23 | 1,5 | 1 | 3 | 80 | 91 |
| 24 | 1 | 1 | 3 | 54 | 96 |
| 25 | 1 | 0,5 | 3 | 16 | n.b. |
| 26 | 1,5 | 0,5 | 3 | 19 | n.b. |
| 27 | 2 | 1 | 3 | 79 | 73 |

| | | | | | |
|---|---|---|---|---|---|
| n.b.: nicht bestimmt | | | | | |

In Beispiel 23 wurde die Reaktionsführung analog Beispiel 21 gewählt, jedoch AlCl₃ einer höheren Reinheit (> 99 %) eingesetzt.

### Vergleichsbeispiel 3

In einem 1I-Kolben mit thermostatiertem Doppelmantel, mechanischer Rührung, Thermometer und Rückflußkühler wurden unter Argonatmosphäre ein Gemisch aus 3,4 mol PCl₃ und 1,2 mol AlCl₃ (98% Reinheit) bei 73 °C vorgelegt. Anschließend wurden innerhalb von 30 min 0,8 mol Fluorbenzol hinzugegeben, wobei ein leichter Argonstrom durch den Reaktionskolben geleitet wurde. Das Reaktionsgemisch wurde 3 Std gerührt, auf 60 °C abgekühlt und 1,25 mol Pyridin innerhalb von 45 min langsam zugegeben. Die Reaktion war exotherm und es entstanden Nebel. Anschließend wurde noch 30 min bei 60 °C weitergerührt. Es fiel ein ungleichmäßiger, großklumpiger Feststoff aus, der nicht über eine Nutsche, sondern lediglich durch Filtration abtrennbar war. Der abfiltrierte Rückstand wurde mit Petrolether gewaschen. Das Filtrat und die Waschflüssigkeit wurde vereinigt und destilliert, wobei man 73,3 g p-Fluorphenyl-dichlorphosphin in einer Ausbeute von 47 % d. Th. erhielt.

## Patentansprüche

1. Verfahren zur Abtrennung von Säuren aus Reaktionsgemischen mittels einer Hilfsbase, **dadurch gekennzeichnet, daß** die Hilfsbase
b) ein Salz mit der Säure bildet, das bei Temperaturen flüssig ist, bei denen das Wertprodukt während der Abtrennung des Flüssigsalzes nicht zersetzt wird und
c) das Salz der Hilfsbase mit dem Wertprodukt oder der Lösung des Wertproduktes in einem geeigneten Lösungsmittel zwei nicht mischbare flüssige Phasen ausbildet, wobei die Säure im Reaktionsverlauf einer Phosphorylierung zur Herstellung von Phosphinen, Phosphinsäureestern, Phosphinigsäureestern (Phosphiniten), Phosphonsäureestern, Phosphonsäurehalogeniden, Phosphonsäureamiden, Phosphonigsäureestern (Phosphoniten), Phosphonigsäureamiden, Phosphonigsäurehalogeniden, Phosphorsäureestern, Phosphorsäurediesterhalogeniden, Phosphorsäurediesteramiden, Phosphorsäureesterdihalogeniden, Phosphorsäureesterdiamiden, Phosphorigsäureesten (Phosphiten), Phosphorigsäurediesterhalogieniden, Phosphorigsäurediesteramiden, Phosphorigsäureesterdihalogeniden freigesetzt wird,
und eine Base eingesetzt wird, entsprechend der Formel (Ie), 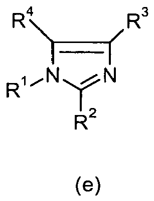
worin
R¹ ausgewählt ist unter Methyl, Ethyl, n-Propyl, n-Butyl, n-Pentyl, n-Octyl, 2-Hydroxyethyl oder 2-Cyanoethyl und
R² bis R⁴ unabhängig voneinander Wasserstoff, Methyl oder Ethyl bedeuten.

2. Verfahren nach Anspruch 1, **dadurch gekennzeichnet, daß** die Hilfsbase zusätzlich d) gleichzeitig als nucleophiler Katalysator fungiert.

3. Verfahren nach einem der Ansprüche 1 bis 2, **dadurch gekennzeichnet, daß** das Salz der Hilfsbase einen Schmelzpunkt unterhalb von 160°C aufweist.

4. Verfahren nach einem der Ansprüche 1 bis 3, **dadurch gekennzeichnet, daß** das Salz der Hilfsbase einen E_{T}(30)-Wert von mehr als 35 aufweist.

5. Verfahren nach einem der Ansprüche 1 bis 4, **dadurch gekennzeichnet, daß** die Hilfsbase 1-n-Butylimidazol oder 1-Methylimidazol ist.

6. Verfahren nach einem der Ansprüche 1 bis 5, **dadurch gekennzeichnet, daß** die Säure Salzsäure, Essigsäure, p-Toluolsulfonsäure, Methansulfonsäure oder Trifluormethansulfonsäure ist.

7. Verfahren nach einem der vorstehenden Ansprüche, **dadurch gekennzeichnet, daß** im abgetrennten Salz der Säure mit der Hilfsbase das molare Verhältnis von Brönsted-Säuren zu Lewis-Säuren größer als 4:1.

8. Verfahren nach einem der Ansprüche 1 bis 7, **dadurch gekennzeichnet, daß** das Salz der Hilfsbase in dem Wertprodukt oder in der Lösung des Wertproduktes in einem geeigneten Lösungsmittel weniger als 20 Gew.-% löslich ist.

9. Verfahren zur Herstellung von Phosphorverbindungen aus den jeweiligen Edukten gemäß Anspruch 1, **dadurch gekennzeichnet, daß** man die Herstellung kontinuierlich bei einer Temperatur von 60°C bis 150°C und einer Verweilzeit unter 1 Stunde durchführt.

10. Verfahren gemäß Anspruch 1, **dadurch gekennzeichnet, daß** die Hilfsbase in nichtionischer Form eingesetzt wird.

## Claims

1. A process for removing acids from reaction mixtures using an auxiliary base, wherein the auxiliary base
b) forms a salt with the acid, said salt being liquid at temperatures at which the product of value does not decompose during the removal of the liquid salt, and
c) the salt of the auxiliary base with the product of value or the solution of the product of value in a suitable solvent forms two immiscible liquid phases, and the acid is released in the course of a phosphorylation for preparation of phosphines, phosphinic esters, phosphinous esters (phosphinites), phosphonic esters, phosphonic halides, phosphonic amides, phosphonous esters (phosphonites), phosphonous amides, phosphonous halides, phosphoric esters, phosphoric diester halides, phosphoric diester amides, phosphoric ester dihalides, phosphoric ester diamides, phosphorous esters (phosphites), phosphorous diester halides, phosphorous diester amides, phosphorous ester dihalides, and a base is used which corresponds to the formula (Ie) 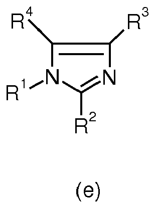
where
R¹ is selected from methyl, ethyl, n-propyl, n-butyl, n-pentyl, n-octyl, 2-hydroxyethyl and 2-cyanoethyl, and
R² to R⁴ are each independently hydrogen, methyl or ethyl.

2. The process according to claim 1, wherein the auxiliary base additionally
d) simultaneously functions as a nucleophilic catalyst.

3. The process according to either of claims 1 and 2, wherein the salt of the auxiliary base has a melting point below 160°C.

4. The process according to any of claims 1 to 3, wherein the salt of the auxiliary base has an E_{T}(30) value of greater than 35.

5. The process according to any of claims 1 to 4, wherein the auxiliary base is 1-n-butylimidazole or 1-methylimidazole.

6. The process according to any of claims 1 to 5, wherein the acid is hydrochloric acid, acetic acid, p-toluenesulfonic acid, methanesulfonic acid or trifluoromethanesulfonic acid.

7. The process according to any of the preceding claims, wherein the molar ratio of Brönsted acids to Lewis acids in the removed salt of the acid with the auxiliary base is greater than 4:1.

8. The process according to any of claims 1 to 7, wherein the salt of the auxiliary base is less than 20% by weight soluble in the product of value or in the solution of the product of value in a suitable solvent.

9. A process for preparing phosphorus compounds from the respective reactants according to claim 1, wherein the preparation is carried out continuously at a temperature of from 60°C to 150°C and a residence time of less than 1 hour.

10. The process according to claim 1, wherein the auxiliary base is used in nonionic form.

## Revendications

1. Procédé pour la séparation d'acides à partir de mélanges réactionnels au moyen d'une base auxiliaire, **caractérisé en ce que** la base auxiliaire
b) forme un sel avec l'acide qui est liquide aux températures auxquelles le produit de valeur n'est pas décomposé pendant la séparation du sel liquide et
c) le sel de la base auxiliaire forme, avec le produit de valeur ou la solution du produit de valeur dans un solvant approprié, deux phases liquides non miscibles, l'acide étant libéré au cours d'une réaction de phosphorylation pour préparer des phosphines, des esters de l'acide phosphinique, des esters de l'acide phosphineux (phosphinites), des esters de l'acide phosphonique, des halogénures de l'acide phosphonique, des amides de l'acide phosphonique, des esters de l'acide phosphoneux (phosphonites), des amides de l'acide phosphoneux, des halogénures de l'acide phosphoneux, des esters de l'acide phosphorique, des diester-halogénures de l'acide phosphorique, des diester-amides de l'acide phosphorique, des ester-dihalogénures de l'acide phosphorique, des ester-diamides de l'acide phosphorique, des esters de l'acide phosphoreux (phosphites), des diester-halogénures de l'acide phosphoreux, des diester-amides de l'acide phosphoreux, des ester-dihalogénures de l'acide phosphoreux, et une base est utilisée, correspondant à la formule (Ie), 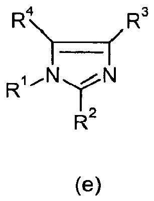
où
R¹ est choisi parmi méthyle, éthyle, n-propyle, n-butyle, n-pentyle, n-octyle, 2-hydroxyéthyle ou 2-cyanoéthyle, et
R² à R⁴ signifient, indépendamment l'un de l'autre hydrogène, méthyle ou éthyle.

2. Procédé selon la revendication 1, **caractérisé en ce que** la base auxiliaire fonctionne en plus d) simultanément comme catalyseur nucléophile.

3. Procédé selon l'une quelconque des revendications 1 à 2, **caractérisé en ce que** le sel de la base auxiliaire présente un point de fusion inférieur à 160°C.

4. Procédé selon l'une quelconque des revendications 1 à 3, **caractérisé en ce que** le sel de la base auxiliaire présente une valeur E_{τ}(30) supérieure à 35.

5. Procédé selon l'une quelconque des revendications 1 à 4, **caractérisé en ce que** la base auxiliaire est le 1-n-butylimidazole ou le 1-méthylimidazole.

6. Procédé selon l'une quelconque des revendications 1 à 5, **caractérisé en ce que** l'acide est l'acide chlorhydrique, l'acide acétique, l'acide p-toluènesulfonique, l'acide méthanesulfonique ou l'acide trifluorométhanesulfonique.

7. Procédé selon l'une quelconque des revendications précédentes, **caractérisé en ce que**, dans le sel de l'acide avec la base auxiliaire séparé, le rapport molaire d'acides de Brönsted à acides de Lewis est supérieur à 4:1.

8. Procédé selon l'une quelconque des revendications 1 à 7, **caractérisé en ce que** le sel de la base auxiliaire dans le produit de valeur ou dans la solution du produit de valeur dans un solvant approprié est soluble à raison de moins de 20% en poids.

9. Procédé pour la préparation de composés phosphorés à partir des différents produits de départ selon la revendication 19, **caractérisé en ce qu'**on réalise la préparation en continu à une température de 60°C à 150°C et pendant un temps de séjour inférieur à 1 heure.

10. Procédé selon la revendication 1, **caractérisé en ce que** la base auxiliaire est utilisée sous forme non ionique.
